# EUROPEAN PATENT APPLICATION

(11) **EP 1 340 501 A1**
(43) Date of publication of application: **03.09.2003**
(21) Application number: 01998197.6
(22) Date of filing: 30.11.2001
(51) Int. Cl.: A61K 31/56, A61K 35/78, A61K 9/20, A61K 9/14, A61P 17/00, A61P 17/16, C07J 63/00, A23D 7/005, A23L 1/30, A23L 1/24, A23L 2/52

(54) **BEAUTIFYING FOODS AND DRINKS AND PERORAL BEAUTIFYING PREPARATIONS**

(30) Priority: 30.11.2000 JP 2000366139
(71) Applicant: The Nisshin Oillio, Ltd., Tokyo 104-0033 (JP)
(72) Inventor: Shinohara, Gou c/o THE NISSHIN OILLIO, LTD.,, Yokosuka-Shi, Kanagawa 239-00832 (JP); Kuno, Noriyasu c/o THhe Isshin Oil Mills, Ltd., Yokosuka-Shi, Kanagawa 239-0832 (JP)
(74) Representative: Bawden, Peter Charles
(86) International application number: JP0110514
(87) International publication number: WO02043736

(57) **Abstract**

The present invention relates to a food or beverage comprising at least one member selected from the group consisting of 5-membered ring-containing triterpenes and physiologically acceptable salts or derivatives thereof. The present invention also relates to an orally administered whitening agent comprising, as an effective component, at least one member selected from the group consisting of 5-membered ring-containing triterpenes and physiologically acceptable salts or derivatives thereof.

## Description

### Background of the Invention

The present invention relates to a food and a beverage, which have an effect of whitening the skin and show their skin-whitening effect when they are orally administered, as well as an orally administered whitening agent.

Important problems concerning beautification of, in particular, women may be those concerning the occurrence of dark skin, liver spots (melasma), ephelis (angel kisses) and dark area (darkening) of the skin and the whitening of the skin has been considered to be more important than before. Each term of liver spots and ephelis used herein is synonymous with freckles. In general, it has been recognized that the dark skin, liver spots, ephelis and dark area of the skin are generated when melanocytes are stimulated by, for instance, the irradiation with UV light rays, any abnormality in the hormone balance and/or any hereditary factor and the melanin pigment bio-synthesized therein is deposited or precipitated in the skin. In the conventionally and principally used method for treating and/or relieving such dark skin, liver spots, ephelis and dark area of the skin, a melanin synthesis-inhibitory agent such as L-ascorbic acid or a derivative thereof, a hydroquinone derivative or a placenta extract is incorporated into a cosmetic and the resulting cosmetic is applied to the skin. In such a cosmetic, however, the effective components thereof are lost with the elapse of time due to, for instance, perspiration and it is thus difficult to sustain the desired effect over a long period of time. Accordingly, the method suffers from such problems that the whitening effect thereof expected is insufficient and that the effect of the components is limited only in the region to which they are applied. More specifically, when the melanin production- inhibitory agent is used in the form of a cosmetic, it should be applied over and over again everyday and it should be applied to the entire body surface on which the dark skin, liver spots, ephelis and dark area of the skin to be treated are occurred or formed and accordingly, this is a severe burden on the users. Moreover, it has also been pointed out that some of the effective components may stimulate the skin and therefore, they are limited in applications; or that some of them give out bad smalls and some of them may undergo precipitation and/or coagulation and accordingly, they are insufficient in the stability; and that some of the effective components may be decomposed or modified due to external stimulations such as exposure to light rays or heat, or any change in the pH value on the surface of the skin through the perspiration and the desired effects thereof are thus deteriorated. In addition, there have been developed orally administered whitening foods and drugs for whitening for the purpose of the elimination of the foregoing drawbacks of the foregoing cosmetics. For instance, there have been known foods of this type, which comprise vitamin C, but these foods suffer from problems such that vitamin C is quite unstable and these foods never show any sufficient whitening effect. Examples of such orally administered products further include a whitening food comprising kojic acid incorporated therein (Japanese Examined Patent Publication (hereunder referred to as "J.P. KOKOKU") Hei 6-16685); a cosmetic, a food and an additive for bath containing a pigmented rice extract (Japanese Un-Examined Patent Publication (hereunder referred to as J.P. KOKAI" Hei 10-287525); an orally administered whitening agent and a food for whitening the skin, which comprise iso-flavone as an effective component (J.P. KOKAI Hei 11-269066); and a whitening food containing pro-anthocyanidin and glutathione (J.P. KOKAI 2000- 60482). Up to now, however, these products may suffer from problems in that they do not show any satisfactory whitening effect and some of the components used therein may have unforeseen side effects encountered when they are orally administered. For this reason, there has presently been desired for the development of a food and a beverage or an orally administered whitening agent, which have sufficient whitening effects and are highly safe.

### Disclosure of the Invention

It is thus an object of the present invention to provide a food and a beverage for whitening the skin and an orally administered whitening agent, which make it possible to continuously enjoy the excellent effect of permitting the prevention or relief of the occurrence or formation of, for instance, the dark skin, liver spots (melasma), ephelis (angel kisses) and dark area (darkening) of the skin, without requiring any prodigious effort like the cosmetics.

The inventors of this invention have conducted various investigations for accomplishing the foregoing object, have found that 5-membered ring-containing triterpenes and physiologically acceptable salts thereof or derivatives thereof possess excellent whitening effects and that one can easily and sufficiently enjoy the whitening effects of these compound through oral administration thereof and have thus completed the present invention.

More specifically, the present invention relates to a food or beverage for whitening the skin or an orally administered whitening agent, which comprises at least one member selected from the group consisting of 5-membered ring-containing triterpenes and physiologically acceptable salts thereof and derivatives thereof. The food or beverage of the present invention possesses a variety of effects, in particular, an effect of whitening the skin. In the present invention, the effective component is used in the form of a food or a beverage, the user can thus easily and continuously ingest the effective component and as a result, it would be expected that goods results could be obtained. Moreover, the orally administered whitening agent of the present invention may be ingested through the oral route without any treatment or may be incorporated into a food or a beverage as a raw material.

The present invention also relates to the foregoing food or beverage in which the 5-membered ring-containing triterpenes and physiologically acceptable salts thereof are isolated from naturally occurring products.

The present invention likewise relates to the foregoing food or beverage or orally administered whitening agent in which the content of the 5-membered ring-containing triterpenes and physiologically acceptable salts thereof or the derivatives thereof is not less than 0.04% by mass on the basis of the total mass of the food or beverage or orally administered whitening agent.

The present invention likewise relates to the foregoing food or beverage or orally administered whitening agent in which the 5-membered ring-containing triterpenes are compounds selected from the group consisting of oleanane type triterpenes, ursane type triterpenes and lupane type triterpenes.

The present invention likewise relates to the foregoing food or beverage or orally administered whitening agent in which the 5-membered ring-containing triterpenes are selected from the group consisting of maslinic acid, erythrodiol, ursolic acid, uvaol, betulinic acid and betulin.

The present invention also relates to the foregoing food or beverage or orally administered whitening agent in which the derivatives of the 5-membered ring- containing triterpenes are alcoholic ester group- or fatty acid ester group-containing derivatives.

The present invention likewise relates to the foregoing food and beverage in which the food and beverage are processed foods.

The present invention also relates to the foregoing food and beverage in which the processed food is an oil and fat preparation or processed oil and fat product.

The present invention also relates to the foregoing food and beverage in which the processed oil and fat product is margarine, shortening, mayonnaise or a dressing.

The present invention likewise relates to the foregoing food and beverage in which the food or beverage is a refreshing beverage.

The present invention also relates to the foregoing food and beverage in which a part or the whole of the 5-membered ring-containing triterpenes are present in the form of physiologically acceptable salts and/or derivatives thereof.

The present invention likewise relates to an edible oil and fat preparation, which comprises maslinic acid prepared by extracting a defatted product derived from olive with an ethanol solution, drying the resulting extract to thus concentrate the same and then purifying the concentrated extract by treating it through chromatography in an amount of not less than 1% by mass.

The present invention also relates to a dressing, which comprises maslinic acid obtained by subjecting olive to oil expression, extracting the resulting oil expression residue with ethanol, drying the resulting extract to thus concentrate the same and then purifying the concentrated extract by treating it through chromatography in an amount of not less than 1% by mass.

The present invention also relates to a tablet-like sweet, which comprises maslinic acid prepared by extracting a defatted product derived from olive with an ethanol solution, drying the resulting extract to thus concentrate the same and then purifying the concentrated extract by treating it through chromatography in an amount of not less than 2.5% by mass.

The present invention also relates to a food or beverage comprising the foregoing orally administered whitening agent incorporated therein.

The present invention also relates to an orally administered whitening agent, which is in the form of a tablet and which comprises maslinic acid prepared by extracting a defatted product derived from olive with an ethanol solution, drying the resulting extract to thus concentrate the same and then purifying the concentrated extract by treating it through chromatography.

The present invention also relates to an orally administered whitening agent, which is in the form of powder and which comprises maslinic acid obtained by subjecting olive to oil expression, extracting the resulting oil expression residue with ethanol, drying the resulting extract to thus concentrate the same and then purifying the concentrated extract by treating it through chromatography.

The present invention also relates to a method of using, as an orally administered whitening agent, at least one compound selected from the group consisting of maslinic acid, erythrodiol, ursolic acid, uvaol, betulinic acid, betulin and physiologically acceptable salts or derivatives thereof.

The present invention also relates to a method of using the foregoing orally administered whitening agent as a prophylactic and/or therapeutic agent for treating dark skin, liver spots (melasma), ephelis (angel kisses), dark area (darkening) of the skin and dullness of the skin.

### Best Mode for Carrying Out the Invention

The present invention relates to a food or beverage for whitening the skin or an orally administered whitening agent, which comprises a compound selected from the group consisting of 5-membered ring-containing triterpenes and physiologically acceptable salts or derivatives thereof.

The food or beverage of the present invention may be, for instance, a variety of foods and beverages such as confectionery, processed foods, oil and fat preparations, milk products and beverages. The shape and properties of the food or beverage of the present invention are not particularly restricted and may be in the solid, half-solid, gel-like, liquid or powdery shapes.

The orally administered whitening agent of the present invention is in general provided in the form of a solid or liquid medicine. More specifically, the orally administered whitening agent of the present invention may be provided in the form of a tablet such as an uncoated tablet, a sugar-coated tablet, a coating tablet, an enteric coated tablet, a chewable tablet, a buccal tablet, a sublingual tablet, a troche tablet or an adhesive tablet; a powder; a capsule such as a hard capsule or a soft capsule; a coated product, a pill, a troche, a liquid preparation, a granule such as a pharmaceutically acceptable sustained release agent thereof, a mixture for internal use, a shake mixture, a suspension, an emulsion, a syrup, a dry syrup, an elixir; and a liquid preparation such as an infusion, a decoction and a limonade, but the present invention is not restricted to these specific examples at all.

The food or beverage of the present invention comprises 5-membered ring-containing triterpenes and accordingly, it has a variety of effects originated from the presence of the triterpenes and the food or beverage is characterized by, in particular, an effect of whitening the skin. The effective components are incorporated into a food or beverage and they can easily and continuously be ingested and it would be expected that they could thus show excellent effect. The 5-membered triterpenes used in the present invention are 5-membered ring-containing compounds among the triterpenes consisting of 6 isoprene units and they are a group of compounds present in a various kinds of plant's bodies in the natural world. These triterpenes can be naturally occurring ones capable of being extracted from plant's bodies, some of them can artificially be synthesized and have already been put on the market as, for instance, reagents and either of these triterpenes can be used in the present invention.

Among the foregoing 5-membered ring-containing triterpenes, those having a whitening effect and preferably used herein are oleanane type triterpenes, ursane type triterpenes and lupane type triterpenes among others and the present invention thus relates to a food or beverage, which comprises at least one member selected from the foregoing triterpenes. Moreover, among the foregoing oleanane type triterpenes, preferred are maslinic acid and/or erythrodiol, with maslinic acid being particularly preferred. Among the foregoing ursane type triterpenes, preferred are ursolic acid and/or uvaol. Among the foregoing lupane type triterpenes, preferred are betulinic acid and/or betulin.

These triterpenes mainly possess effects of whitening the skin, the whitening effect thereof can be evaluated by the continuous ingestion of the same, but the effect can likewise be evaluated by a test, which makes use of cultured pigment cells. According to this evaluation method, it has been confirmed that these triterpenes have excellent whitening effects on the order of several ten times to several hundred times the whitening effect observed for vitamin C and derivatives thereof as conventionally known orally administered whitening agents.

In the food or beverage of the present invention, the content of at least one member selected from the group consisting of 5-membered ring-containing triterpenes and physiologically acceptable salts or derivatives thereof may vary depending on the ingestion frequency and ingested amount of the food or beverage and other conditions and therefore, it can appropriately be controlled and is not particularly restricted, but the content thereof can be adjusted to the range of from 0.00001 to 50% by mass.

In this respect, examples of foods and beverages are confectionery, processed foods, oil and fat preparations, milk products and beverages and the shape and properties thereof are not particularly restricted and may be in the solid, half-solid, gel-like, liquid or powdery shapes, as has been described above. The foregoing 5-membered ring-containing triterpenes and/or specific derivatives thereof are, on the whole, oil-soluble compounds and accordingly, particularly preferred shapes of the foods and beverages in this case are oil and fat preparations and/or processed oil and fat products. Specific examples thereof include, but are not particularly restricted to oil and fat preparations having high maslinic acid contents. Moreover, preferred examples of such foods and beverages also include those obtained by using the oil and fat preparations and/or processed oil and fat products as raw materials or those obtained by the use thereof as fried foods or fries.

Furthermore, aqueous foods and beverages such as beverages, for instance, refreshing drinks can be prepared as the foods or beverages of the present invention. In particular, a part or the whole of the 5-membered ring-containing triterpenes may preferably be replaced with physiologically acceptable salts and/or specific derivatives thereof since the substitution permits the improvement of the water-solubility of the 5-membered ring-containing triterpenes, which are, by nature, oil-soluble as a whole. Examples of such foods and beverages include, but are not particularly restricted to, aqueous foods and beverages such as refreshing drinks containing incorporated physiologically acceptable salts or derivatives of maslinic acid, which is, by nature, oil-soluble.

In addition, the present invention likewise relate to an orally administered whitening agent, which comprises, as an effective component, at least one member selected from the group consisting of 5-membered ring-containing triterpenes and physiologically acceptable salts or derivatives thereof, as has been discussed above. The orally administered whitening agent according to the present invention may be orally ingested without any pre-treatment or may be incorporated into foods and beverages as ingredients thereof.

In the preferred embodiment of the foregoing orally administered whitening agent, the foregoing 5-membered ring-containing triterpenes are at least one member selected from the group consisting of oleanane type triterpenes, ursane type triterpenes and lupane type triterpenes. Moreover, among the foregoing oleanane type triterpenes, preferred are maslinic acid and/or erythrodiol, with maslinic acid being particularly preferred. Among the foregoing ursane type triterpenes, preferred are ursolic acid and/or uvaol. Among the foregoing lupane type triterpenes, preferred are betulinic acid and/or betulin.

In this respect, the passage "comprising, as an effective component" herein used means that the compound is included in such an amount that the compound can show the desired effect such as a whitening effect, but the content is not restricted to any specific range and may appropriately be adjusted depending on the ingestion frequency, the ingested amount and the purpose of use. For instance, when the agent is directly ingested through the oral route, the effective component may be used in a relatively low concentration, while if using the agent as, for instance, an ingredient for foods and beverages, it is preferably used in a high concentration.

As has been described above, a food or beverage having an effect of whitening the skin can be prepared by incorporating the orally administered whitening agent according to the present invention as a raw material.

The present invention also relates to a method for using, as an orally administered whitening agent, at least one member selected from the group consisting of maslinic acid and erythrodiol as oleanane type triterpenes; ursolic acid and uvaol as ursane type triterpenes; betulinic acid and betulin as lupane type triterpenes; and physiologically acceptable salts or derivatives thereof.

As has been discussed above, the present invention relates to a food or beverage for whitening the skin or an orally administered whitening agent, which comprises a compound selected from the group consisting of 5-membered ring-containing triterpenes and physiologically acceptable salts or derivatives thereof. In this respect, the term "5-membered ring-containing triterpenes" herein used means a kind of triterpenes and 5-membered ring-containing compounds each comprising 6 isoprene units. The carbon atom number thereof is basically 30, but may be higher or lower than 30 since the number of carbon atoms may be changed through rearrangement, oxidation, elimination or alkylation during the biosynthesis process therefor. The "5-membered ring-containing triterpenes" used in the present invention include, for instance, the foregoing 5-membered ring-containing triterpenes, physiologically acceptable salts thereof and/or derivatives obtained by substituting the hydroxyl and/or carboxyl groups thereof with other substituents.

These compounds may be obtained from naturally occurring plants, may artificially be prepared or those commercially available may likewise suitably be used.

The 5-membered ring-containing triterpenes are in general classified on the basis of the skeletons thereof. The present invention is not restricted to any specific 5-membered ring-containing triterpene, but examples thereof include oleanane type triterpenes, ursane type triterpenes, lupane type triterpenes, hopane type triterpenes, seratane type triterpenes, friedelane type triterpenes, taraxerane type triterpenes, taraxastane type triterpenes, multiflorane type triterpenes and germanicane type triterpenes.

In this connection, the "physiologically acceptable salt" herein used means, in particular, salts derived from the carboxyl group of the 5-membered ring-containing triterpenoic acid (partial structure: -COOX; X represents an arbitrary cationic substance) and also includes those included, by nature, in isolates derived from natural products. In the present invention, such salts are not particularly restricted inasmuch as they are currently used in foods and beverages or pharmaceutical compositions and specific examples thereof are alkali metal salts such as sodium, potassium and lithium salts; alkaline earth metal salts such as calcium, magnesium, barium and zinc salts; alkylamine salts such as ammonium, methylamine, dimethylamine, trimethylamine, ethylamine, diethylamine, triethylamine, propylamine, butylamine, tetrabutylamine, pentylamine and hexylamine salts; alkanolamine salts such as ethanolamine, diethanolamine, triethanolamine, propanolamine, dipropanolamine, isopropanolamine and diisopropanolamine salts; salts with other organic amines such as piperazine and piperidine; and salts with basic amino acids such as lysine, alginine, histidine and tryptophane. Among these salts, preferred are alkali metal salts, alkylamine salts, alkanolamine salts and basic amino acid salts. These salts in general have water-solubility higher than those observed for the original 5-membered ring-containing triterpenes and therefore, these salts are preferably used, in particular, in aqueous foods and beverages in the present invention.

Moreover, the term "derivative(s)" herein used means those capable of being biochemically or artificially formed and the present invention is not restricted to specific ones inasmuch as they can practically be formed. Examples thereof include derivatives each having an alcohol ester group, derivatives each carrying a fatty acid ester group, those each having an alkoxy group, those each having an alkoxymethyl group or glycosides. Among these, the derivatives each having an alcohol ester group, derivatives each carrying a fatty acid ester group, those each having an alkoxy group and those each having an alkoxymethyl group have, in particular, oil-solubility higher than those observed for the original 5-membered ring-containing triterpenes and therefore, they are preferably used, in particular, in oil-based foods and beverages, while the glycosides have higher water-solubility as compared with the original 5-membered ring-containing triterpenes and therefore, they are preferably used, in particular, in aqueous foods and beverages, in the present invention.

A part of these derivatives are also present in nature or may artificially be formed as has been discussed above. Moreover, the derivatives of the present invention are again derivatized and the salts thereof may be used herein.

As has been discussed above, the water-solubility or oil-solubility of the 5-membered ring-containing triterpenes may be improved by converting the triterpenes into physiologically acceptable and suitable salts or derivatives thereof and therefore, it is possible to design any product whose handling ability, quality and whitening effect are improved.

The term "alcohol ester group" herein used means a functional group formed as a result of a general dehydration reaction between a carboxyl group and an alcohol (partial structure: -COOR; R represents an arbitrary hydrocarbon type functional group). In other words, the derivative of a 5-membered ring-containing triterpene carrying an alcohol ester group used in the present invention, in particular, means one capable of being formed from the carboxyl group thereof with an alcohol. In this respect, the alcohol is not restricted to any specific one, but specific examples thereof are methanol, ethanol, n-propanol, iso-propanol, allyl alcohol, n-butanol, sec-butanol, tert-butanol, ethylene glycol, trimethylsilyl alcohol, triethylsilyl alcohol, phenol, benzyl alcohol and saccharides. Among these, preferred are derivatives derived from ethanol, triethylsilyl alcohol, methanol, n-propanol, iso-propanol and trimethylsilyl alcohol.

The term "fatty acid ester group" used herein means a functional group formed as a result of a general dehydration reaction between a hydroxyl group and a fatty acid (partial structure: -OCOR; R represents an arbitrary hydrocarbon type functional group). In other words, the derivative of a 5-membered ring-containing triterpene carrying a fatty acid ester group used in the present invention, in particular, means one capable of being formed from the hydroxyl group thereof with a fatty acid. In this case, the fatty acid is not restricted to any particular one, but specific examples thereof include acetic acid, acetic anhydride, propionic acid, butyric acid, isobutyric acid, valeric acid, iso-valeric acid, pivalic acid, caproic acid, caprylic acid, capric acid, undecanoic acid, lauric acid, myristic acid, palmitic acid, palmitoleic acid, stearic acid, oleic acid, elaidic acid, vaccenic acid, linoleic acid, linoelaidic acid, linolenic acid, γ-linolenic acid, arachidic acid, arachidonic acid, eicosapentaenoic acid, behenic acid, docosahexaenoic acid, lignoceric acid, cerotic acid, montanic acid and melissic acid. Among these, preferably used herein are derivatives derived from acetic acid, acetic anhydride, caproic acid, caprylic acid, capric acid, lauric acid, myristic acid, palmitic acid, palmitoleic acid, stearic acid, oleic acid, elaidic acid, linoleic acid, linoelaidic acid, linolenic acid, γ-linolenic acid, arachidic acid, arachidonic acid, eicosapentaenoic acid, behenic acid and docosahexaenoic acid.

The term "alkoxy group" used herein means a functional group formed as a result of a general dehydration reaction between a hydroxyl group and an alcohol (partial structure: -OR; R represents an arbitrary hydrocarbon type functional group). In other words, the derivative of a 5-membered ring-containing triterpene carrying an alkoxy group used in the present invention, in particular, means one capable of being formed from the hydroxyl group thereof with an alcohol. In this case, the alcohol is not restricted to any particular one, but specific examples thereof include methanol, ethanol, n-propanol, iso-propanol, allyl alcohol, n-butanol, sec-butanol, tert-butanol, ethylene glycol, trimethylsilyl alcohol, triethylsilyl alcohol, phenol, benzyl alcohol and saccharides. Among these, preferred are derivatives derived from ethanol, triethylsilyl alcohol, methanol, n-propanol, iso-propanol and trimethylsilyl alcohol.

The term "alkoxymethyl group" used herein means a functional group formed as a result of a general dehydration reaction between a hydroxymethyl group and an alcohol (partial structure: - CH₂OR; R represents an arbitrary hydrocarbon type functional group). In other words, the derivative of a 5-membered ring-containing triterpene carrying an alkoxymethyl group used in the present invention, in particular, means one capable of being formed from the hydroxymethyl group thereof with an alcohol. In this case, the alcohol is not restricted to any particular one, but specific examples thereof include methanol, ethanol, n-propanol, iso-propanol, allyl alcohol, n-butanol, sec-butanol, tert-butanol, ethylene glycol, trimethylsilyl alcohol, triethylsilyl alcohol, phenol, benzyl alcohol and saccharides. Among these, preferred are derivatives derived from ethanol, triethylsilyl alcohol, methanol, n-propanol, iso-propanol and trimethylsilyl alcohol.

Moreover, the term "glycosides" herein used means, in particular, derivatives capable of being formed from the carboxyl group, hydroxyl group and/or hydroxymethyl group of the 5-membered ring-containing triterpenes with saccharides among the foregoing alcohol ester group-containing derivatives, alkoxy group-containing derivatives and alkoxymethyl group-containing derivatives (partial structure: -COOR, -OR, -CH₂OR; R represents an arbitrary sugar residue). In this respect, the saccharides are not restricted to particular ones, but specific examples thereof are glucose, mannose, galactose, fructose, xylose, arabinose, fucose, rhamnose, glucosamine, galactosamine and glucuronic acid, wherein each of these saccharides may be either α-isomer or β-isomer. Moreover, these glycosides may be monosaccharides or any oligo saccharides comprising various combinations of disaccharides and/or higher saccharides or polysaccharides. Among these, some glycosides in general exist in nature and have been known under the common name of "saponin", but either of them can be used in the present invention.

The 5-membered ring-containing triterpenes used in the present invention are those described above, but preferred are, in particular, oleanane type, ursane type, and lupane type triterpenes and physiologically acceptable salts or derivatives thereof from the viewpoint of the intensity of their whitening effect. The oleanane type triterpenes and physiologically acceptable salts or derivatives thereof have skeletal structures represented by the following general formula (I), the ursane type triterpenes and physiologically acceptable salts or derivatives thereof have skeletal structures represented by the following general formula (II) and the lupane type triterpenes and physiologically acceptable salts or derivatives thereof have skeletal structures represented by the following general formula (III). Moreover, the functional groups in each formula are the same as those specified above. 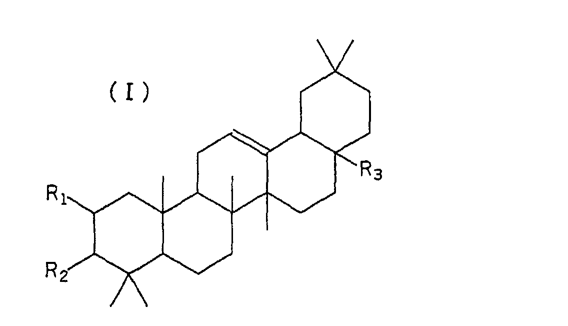 (In the formula, R₁ and R₂ each represents a hydrogen atom (-H), a hydroxyl group (-OH), an alkoxy group (-OR) or an alcohol ester group (-OCOR); and R₃ represents a methyl group (-CH₃), a hydroxymethyl group (-CH₂OH), an alkoxymethyl group (-CH₂OR), a carboxyl group (-COOH), a fatty acid ester group (-COOR) or a carboxylic acid salt residue (-COOX)). 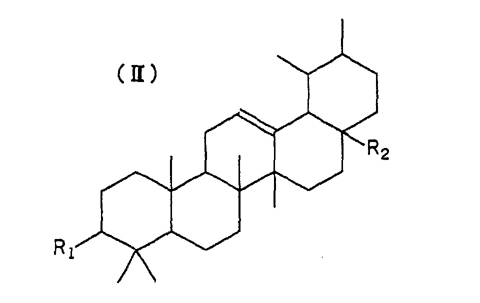 (In the formula, R₁ represents a hydrogen atom (-H), a hydroxyl group (-OH), an alkoxy group (-OR) or an alcohol ester group (-OCOR); and R₂ represents a methyl group (-CH₃), a hydroxymethyl group (-CH₂OH), an alkoxymethyl group (-CH₂OR), a carboxyl group (-COOH), a fatty acid ester group (-COOR) or a carboxylic acid salt residue (-COOX)). 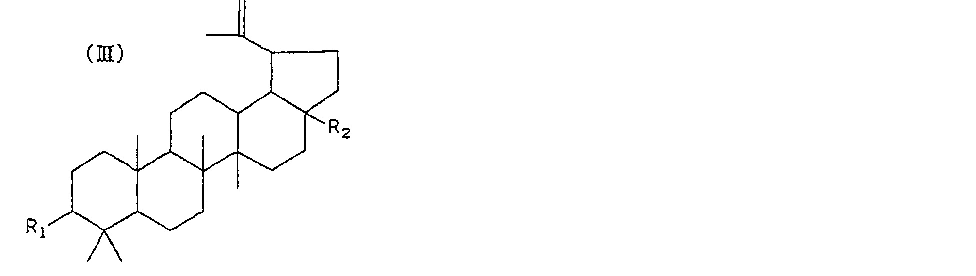 (In the formula, R₁ represents a hydrogen atom (-H), a hydroxyl group (-OH), an alkoxy group (-OR) or an alcohol ester group (-OCOR); and R₂ represents a methyl group (-CH₃), a hydroxymethyl group (-CH₂OH), an alkoxymethyl group (-CH₂OR), a carboxyl group (-COOH), a fatty acid ester group (-COOR) or a carboxylic acid salt residue (-COOX)).

These triterpenes usable in the present invention are not restricted to specific ones, but specific examples of oleanane type triterpenes include maslinic acid, oleanolic acid, erythrodiol, β-amyrin, hederagenin, and glycyrrhetic acid; specific examples of ursane type triterpenes are ursolic acid, uvaol, α -amyrin, quinovic acid, taraxasterol and α-hydroxy-ursolic acid; and specific examples of lupane type triterpenes include betulinic acid, betulin and lupeol. In addition, the physiologically acceptable salts and derivatives thereof are the same as those described above. When using physiologically acceptable salts or derivatives thereof, the oleanane type, ursane type and lupane type triterpenes and physiologically acceptable salts or derivatives thereof are not limited in their sources and they may be those derived from naturally occurring substances, those artificially synthesized and commercially available ones. However, it is quite preferred to use those derived from natural substances while taking into consideration the fact that these triterpenes are administered through the oral route.

As has been discussed above, 5-membered ring-containing triterpenes preferably used in the present invention are oleanane type triterpenes represented by Formula (I), ursane type triterpenes represented by Formula (II), lupane type triterpenes represented by Formula (III) and physiologically acceptable salts or derivatives thereof, but more preferably used herein are oleanane type triterpenes such as maslinic acid and erythrodiol; ursane type triterpenes such as ursolic acid and uvaol; and lupane type triterpenes such as betulinic acid and betulin from the viewpoint of the intensity of the whitening effect. In this connection, it is a matter of course that physiologically acceptable salts or derivatives of the foregoing triterpenes can likewise be preferably used in the present invention.

Both maslinic acid and erythrodiol are oleanane type triterpenes and it has been known that they are present in a variety of plants. Moreover, the physiologically acceptable salts and derivatives thereof are the same as those described above. When using maslinic acid, erythrodiol, physiologically acceptable salts thereof or derivatives thereof in the food or beverage and orally administered whitening agent of the present invention, the sources thereof are not restricted to specific ones and these substances may be those derived from natural resources, artificially synthesized ones and commercially available ones, but those derived from naturally occurring substances are preferably used herein while taking into consideration the fact that these triterpenes are administered through the oral route.

In the present invention, most preferably used are maslinic acid and/or physiologically acceptable salts thereof in the light of the intensity of the resulting whitening effect and stable supply. Maslinic acid is a kind of oleanane type triterpenes, represented by the following chemical formula (IV) and it has been known that they possess functions such as anti-inflammatory effect and anti-histamic effect. It is also known that they are present in, for instance, natural plants such as olive, hop, mint, pomegranate, clove, sage and jujube, in nature. In the food or beverage and orally administered whitening agent according to the present invention, the sources of maslinic acid and/or physiologically acceptable salts thereof are not restricted to specific ones and may be those derived from natural substances, artificially synthesized ones and commercially available ones. However, preferably used herein are those derived from natural resources such as olive, hop, mint, pomegranate, clove, sage and jujube and, in particular, maslinic acid and/or physiologically acceptable salts thereof derived from olive are quite preferred because of the stable supply of the raw material and high content. Maslinic acid and/or physiologically acceptable salts thereof can be obtained by extracting these raw materials, in particular, olive plant and/or products obtained in the olive oil-manufacture processes with water and/or an organic solvent and the resulting extract can further be concentrated and/or purified to easily give highly concentrated naturally occurring maslinic acid and/or physiologically acceptable salts thereof in a large amount.

In the specification, the term "olive" means olive plant and/or olive oil and/or products obtained in the olive oil-manufacture processes.

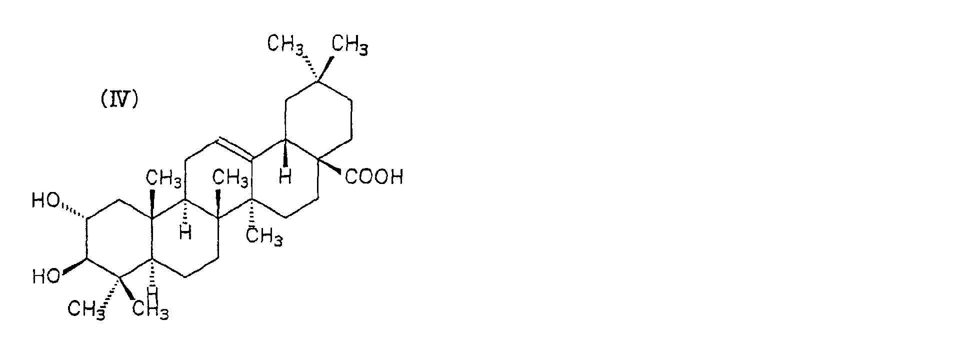

In the present invention, the physiologically acceptable salts or derivatives of maslinic acid are the same as those described above. More specifically, the physiologically acceptable salts of maslinic acid are salts derived from the -COOH group in the chemical formula (IV) and the kinds of the salts are not restricted to specific ones inasmuch as they may be commonly used in foods and beverages or pharmaceutical compositions. Specific examples of the maslinic acid salts are sodium maslinate,- potassium maslinate, ammonium maslinate, dimethylammonium maslinate, calcium maslinate and magnesium maslinate, with sodium maslinate and potassium maslinate being preferred among others.

Moreover, examples of derivatives of maslinic acid are those derivatized at any one position such as methyl maslinate, ethyl maslinate, n-propyl maslinate, isopropyl maslinate, n-butyl maslinate, trimethylsilyl maslinate, triethylsilyl maslinate, maslinic acid-β-D-glucopyranosyl ester, maslinic acid-β-D-galactopyranosyl ester, 3-O-acetyl- maslinic acid, 3-O-propionyl-maslinic acid, 3-O-butyryl-maslinic acid, 3-O-valelyl- maslinic acid, 3-O-capryl-maslinic acid, 3-O-lauryl-maslinic acid, 3-O-myristyl-maslinic acid, 3-O-palmityl-masloinic acid, 3-O-palmitooleyl-maslinic acid, 3-O-stearyl-maslinic acid, 3-O-stearoyl-maslinic acid, 3-O-oleyl-maslinic acid, 3-O-vaccenyl-maslinic acid, 3-O-linoleyl-maslinic acid, 3-O-linolenyl-maslinic acid, 3-O-arachidyl-maslinic acid, 3-O-arachidonyl-maslinic acid, 3-O-behenyl-maslinic acid, 2-O-acetyl-maslinic acid, 2-O-propionyl-maslinic acid, 2-O-butyryl-maslinic acid, 2-O-valelyl-maslinic acid, 2-O-capryl-maslinic acid, 2-O-lauryl-maslinic acid, 2-O-myristyl-maslinic acid, 2-O-palmityl-masloinic acid, 2-O-palmitooleyl-maslinic acid, 2-O-stearyl-maslinic acid, 2-O-stearoyl-maslinic acid, 2-O-oleyl-maslinic acid, 2-O-vaccenyl-maslinic acid, 2-O-linoleyl-maslinic acid, 2-O-linolenyl-maslinic acid, 2-O-arachidyl-maslinic acid, 2-O-arachidonyl-maslinic acid, 2-O-behenyl-maslinic acid, 3-O-methyl-maslinic acid, 3-O-ethyl-maslinic acid, 3-O-t-butyl-maslinic acid, 3-O-triethylsilyl-maslinic acid, 3-O- β -D-glucopyranosyl-maslinic acid, 3-O- β -D-galactopyranosyl-maslinic acid, 3-O- β -D- glucuronopyranosyl-maslinic acid, 2-O-methyl-maslinic acid, 2-O-ethyl-maslinic acid, 2-O-t-butyl-maslinic acid, 2-O-triethylsilyl-maslinic acid, 2-O- β -D-glucopyranosyl- maslinic acid, 2-O- β -D-galactopyranosyl-maslinic acid and 2-O- β -D-glucurono-pyranosyl-maslinic acid. Among these derivatives, preferred are ethyl maslinate, triethylsilyl maslinate, 3-O-acetyl-maslinic acid, 2-O-acetyl-maslinic acid, 2-O- triethylsilyl-maslinic acid, 3-O-stearoyl-maslinic acid and 2-O-stearoyl-maslinic acid. The foregoing are derivatives derivatized only at one position, but the derivatives usable in the present invention may be those derivatized at two or more possible positions or at two or more groups. Examples thereof preferably used herein are 2,3-O-diacetyl derivatives, 2,3-O-di-triethylsilyl derivatives and 2,3-di-stearoyl derivatives of maslinic acid or the foregoing preferred maslinic acid esters. Moreover, only derivatives with monosaccharides are listed above as glycosides, but it is a matter of course that the saccharides may be oligosaccharides higher than disaccharides comprising a variety of saccharides.

Erythrodiol is a kind of oleanane type triterpenes, has a structure represented by the following chemical formula (V) and up to this time, it has been known that this compound possesses an anti-inflammatory effect (Planta. Med., Vol. 61, No. 2, pp. 182-185, 1995). It has been known that this compound is present in nature and exists in, for instance, olive, sunflower, common marigold, gum Arabic tree, and red sanders. In the food or beverage and orally administered whitening agent of the present invention, the sources of erythrodiol or derivatives thereof are not restricted to specific ones and they may be those derived from naturally occurring substances, artificially synthesized ones or commercially available ones, but preferably used herein are those derived from natural resources such as olive, sunflower, common marigold, gum Arabic tree, and red sanders, while taking into consideration the fact that they are ingested through the oral route. In particular, preferred are those derived from olive and more specifically, those obtained from olive plant and/or products obtained in the olive oil-manufacture processes. 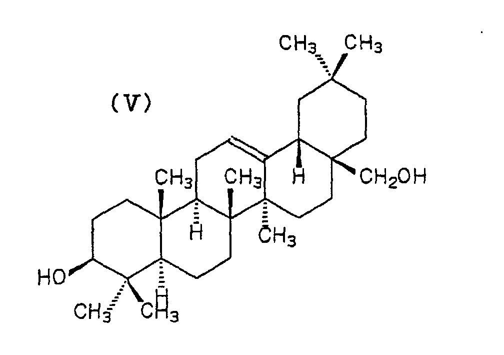 The physiologically acceptable salts and derivatives of erythrodiol are the same as those described above.

Examples of derivatives of erythrodiol derivatized only at one position include, but are not restricted to, 3-O-acetyl-erythrodiol, 3-O-propionyl-erythrodiol, 3-O-butyryl-erythrodiol, 3-O-valeryl-erythrodiol, 3-O-capryl-erythrodiol, 3-O-lauryl-erythrodiol, 3-O-myristyl-erythrodiol, 3-O-palmityl-erythrodiol, 3-O-palmitooleyl-erythrodiol, 3-O- stearyl-erythrodiol, 3-O-oleyl-erythrodiol, 3-O-vaccenyl-erythrodiol, 3-O-linoleyl-erythrodiol, 3-O-linolenyl-erythrodiol, 3-O-arachidyl-erythrodiol, 3-O-arachidonyl- erythrodiol, 3-O-behenyl-erythrodiol, 28-O-acetyl-erythrodiol, 28-O-propionyl-erythrodiol, 28-O-butyryl- erythrodiol, 28-O-valeryl-erythrodiol, 28-O-capryl-erythrodiol, 28-O-lauryl-erythrodiol, 28-O-myristyl-erythrodiol, 28-O-palmityl-erythrodiol, 28-O-palmito-oleyl-erythrodiol, 28-O-stearyl-erythrodiol, 28-O-oleyl-erythrodiol, 28-O-vaccenyl-erythrodiol, 28-O-linoleyl-erythrodiol, 28-O-linolenyl-erythrodiol, 28-O-arachidyl-erythrodiol, 28-O-arachidonyl-erythrodiol, 28-O-behenyl-erythrodiol, 3-O- methyl-erythrodiol, 3-O-ethyl-erythrodiol, 3-O-t-butyl-erythrodiol, 3-O-triethylsilyl-erythrodiol, 28-O-methyl-erythrodiol, 28-O-ethyl-erythrodiol, 28-O-t-butyl-erythrodiol, 28-O-triethylsilyl-erythrodiol, 3-O- β -D-glucopyranosyl-erythrodiol, 3-O-β -D-galacto- pyranosyl-erythrodiol, 3-O- β -D-glucuronopyranosyl-erythrodiol, 28-O- β -D-gluco-pyranosyl-erythrodiol, 28-O- β -D-galactopyranosyl-erythrodiol and 28-O- β -D- glucuronopyranosyl-erythrodiol. Among these derivatives, preferred are 3-0-acetyl- erythrodiol and 28-O-acetyl-erythrodiol. The foregoing are derivatives derivatized only at one position or group, but the derivatives usable in the present invention may be those derivatized at two or more possible positions or two or more groups. Examples thereof include 3,28-O-diacetyl-erythrodiol. Moreover, only derivatives with monosaccharides are listed above as glycosides, but it is a matter of course that the saccharides may be oligosaccharides higher than disaccharides comprising a variety of saccharides.

Both ursolic acid and uvaol are ursane type triterpenes and it has been known that these substances are present in a variety of plants. Moreover, physiologically acceptable salts and derivatives thereof are the same as those discussed above. When using ursolic acid, uvaol, physiologically acceptable salts thereof or derivatives thereof in the foods or beverages and orally administered whitening agent of the present invention, the sources of these substances are not restricted to specific ones at all and may be those derived from natural resources, artificially synthesized ones or commercially available ones, but preferably used herein are those derived from naturally occurring substances while taking into consideration the fact that they are ingested through the oral route.

Ursolic acid is a kind of ursane type triterpenes, is a compound having the structure represented by the following chemical formula (VI) and, up to this time, it has been known that this compound possesses an anti-inflammatory effect, an anti-arteriosclerotic action, an anti-diabetic action and anti-lipemic action (Jie Liu, Journal of Ethnopharmacology, 1995, 49: 57-68). It has been known that ursolic acid is widely distributed in nature and that it exists in, for instance, fruits and/or leaves of plants such as apple, cherry tree and bearberry. In the foods or beverages and orally administered whitening agent of the present invention, the sources of ursolic acid and physiologically acceptable salts or derivatives thereof are not restricted to specific ones and may be those derived from natural resources, artificially synthesized ones or commercially available ones, but preferably used herein are those derived from naturally occurring substances such as apple, cherry tree and bearberry while taking into consideration the fact that they are ingested through the oral route.

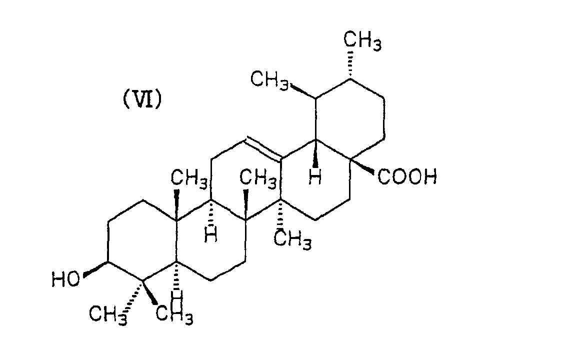

Regarding ursolic acid, physiologically acceptable salts and derivatives thereof are the same as those discussed above.

Examples of physiologically acceptable salts of ursolic acid include, but are not restricted to, sodium ursolate, potassium ursolate, ammonium ursolate, dimethyl- ammonium ursolate, calcium ursolate and magnesium ursolate.

Examples of derivatives of ursolic acid, which are derivatized only at one position, include ursolic acid methyl ester, ursolic acid ethyl ester, ursolic acid n-propyl ester, ursolic acid isopropyl ester, ursolic acid n-butyl ester, ursolic acid trimethylsilyl ester, ursolic acid triethylsilyl ester, ursolic acid β -D-glucopyranosyl ester, ursolic acid β-D-galactopyranosyl ester, 3-O-acetyl-ursolic acid, 3-O-propionyl-ursolic acid, 3-O-butyryl- ursolic acid, 3-O-valeryl-ursolic acid, 3-O-capryl-ursolic acid, 3-O-lauryl-ursolic acid, 3-O-myristyl-ursolic acid, 3-O-palmityl-ursolic acid, 3-O-palmito-oleyl-ursolic acid, 3-O-stearyl-ursolic acid, 3-O-oleyl-ursolic acid, 3-O-vaccenyl-ursolic acid, 3-O-linoleyl- ursolic acid, 3-O-linolenyl-ursolic acid, 3-O-arachidyl-ursolic acid, 3-O-arachidonyl- ursolic acid, 3-O-behenyl-ursolic acid, 3-O-methyl-ursolic acid, 3-O-ethyl-ursolic acid, 3-O-t-butyl-ursolic acid, 3-O-triethylsilyl-ursolic acid, 3-O-β-D-glucopyranosyl-ursolic acid, 3-O-β-D-galactopyranosyl-ursolic acid and 3-O-β-D-glucuronopyranosyl-ursolic acid. The foregoing are derivatives derivatized only at one position or group, but the derivatives usable in the present invention may be those derivatized at two or more possible positions or at two or more groups. Moreover, only derivatives with monosaccharides are listed above as glycosides, but it is a matter of course that the saccharides may be oligosaccharides higher than disaccharides comprising a variety of saccharides.

Uvaol is a kind of ursane type triterpenes, is a compound having the structure represented by the following chemical formula (VII) and, up to this time, it has been known that this compound possesses, for instance, an anti-inflammatory effect (Planta. Med., Vol. 61, No. 2, pp. 182-185, 1995) and a glycerophosphate dehydrogenase- inhibitory effect (J.P. KOKAI Hei 9-67249). It has been known that this compound is present in nature and exists in, for instance, olive, bearberry, sage, gum Arabic tree and punk tree. In the foods or beverages and orally administered whitening agent of the present invention, the sources of uvaol or derivatives thereof are not restricted to specific ones and may be those derived from natural resources, artificially synthesized ones or commercially available ones, but preferably used herein are those derived from naturally occurring substances such as olive, bearberry, sage, gum Arabic tree and punk tree while taking into consideration the fact that they are ingested through the oral route. In particular, olive is preferably used and more specifically, preferably used herein are those derived from olive plant and/or products obtained in the olive oil-manufacture processes.

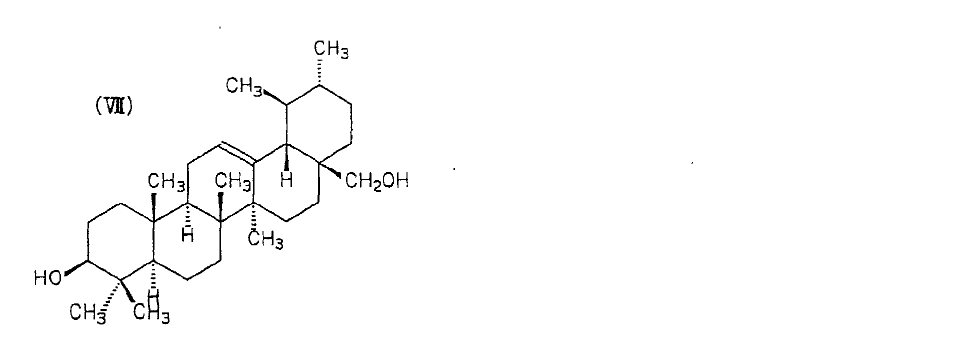

In respect of uvaol, the physiologically acceptable salts and derivatives thereof are the same as those described above.

Examples of derivatives of uvaol, which are derivatized only at one position, include, but are not restricted to, 3-O-acetyl-uvaol, 3-O-propionyl-uvaol, 3-O-butyryl- uvaol, 3-O-valeryl-uvaol, 3-O-capryl-uvaol, 3-O-lauryl-uvaol, 3-O-myristyl-uvaol, 3-O- palmityl-uvaol, 3-O-palmito-oleyl-uvaol, 3-O-stearyl-uvaol, 3-O-oleyl-uvaol, 3-O-vaccenyl-uvaol, 3-O-linoleyl-uvaol, 3-O-linolenyl-uvaol, 3-O-arachidyl-uvaol, 3-O- arachidonyl-uvaol, 3-O-behenyl-uvaol, 28-O-acetyl-uvaol, 28-O-propionyl-uvaol, 28-O- butyryl-uvaol, 28-O-valeryl-uvaol, 28-O-capryl-uvaol, 28-O-lauryl-uvaol, 28-0-myristyl-uvaol, 28-O-palmityl-uvaol, 28-O-palmito-oleyl-uvaol, 28-O-stearyl-uvaol, 28-O-oleyl- uvaol, 28-O-vaccenyl-uvaol, 28-O-linoleyl-uvaol, 28-O-linolenyl-uvaol, 28-O-arachidyl- uvaol, 28-O-arachidonyl-uvaol, 28-O-behenyl-uvaol, 3-O-methyl-uvaol, 3-O-ethyl-uvaol, 3-O-t-butyl-uvaol, 3-O-triethylsilyl-uvaol, 28-O-methyl-uvaol, 28-O-ethyl-uvaol, 28-O-t- butyl-uvaol, 28-O-triethylsilyl-uvaol, 3-O- β -D-glucopyranosyl-uvaol, 3-O- β -D-galacto- pyranosyl-uvaol, 3-O- β-D-glucuronopyranosyl-uvaol, 28-O-β-D-glucopyranosyl-uvaol, 28-O-β-D-galactopyranosyl-uvaol and 28-O- β -D-glucuronopyranosyl-uvaol. The foregoing are derivatives derivatized only at one position or group, but the derivatives usable in the present invention may be those derivatized at two or more possible positions or with two or more groups. Examples thereof include 3,28-O-diacetyl-uvaol. Moreover, only derivatives with monosaccharides are listed above as glycosides, but it is a matter of course that the saccharides may be oligosaccharides higher than disaccharides comprising a variety of saccharides.

Both betulinic acid and betulin are lupane type triterpenes and it has been known that they are present in a variety of plants. The physiologically acceptable salts and derivatives thereof are the same as those described above. When using betulinic acid, betulin, physiologically acceptable salts or derivatives thereof in the foods or beverages and orally administered whitening agent of the present invention, the sources of these substances are not restricted to specific ones and may be those derived from natural resources, artificially synthesized ones or commercially available ones, but preferably used herein are those derived from naturally occurring substances while taking into consideration the fact that they are ingested through the oral route.

Betulinic acid is a kind of lupane type triterpenes, has a structure represented by the following chemical formula (VIII) and up to this time, it has been known that this compound possesses various effects such as an anticancer effect, an anti-inflammatory effect and a wound-healing promotion effect (J.P. KOKOKU Hei 4-26623), an alcohol absorption-inhibitory effect (J.P. KOKAI Hei 7-53385) and a new hair growth-promoting effect (J.P. KOKAI Hei 9-157139). It has been known that, in nature, betulinic acid is present in Japanese green gentian, clove, the rind of grapes and olive in its free state and in Panax japonicus C. A. Meyer, carrot and sugar beet in the form of saponin. In the foods or beverages and orally administered whitening agent of the present invention, the sources of betulinic acid and physiologically acceptable salts or derivatives thereof are not restricted to specific ones and may be those derived from natural resources, artificially synthesized ones or commercially available ones, but preferably used herein are those derived from naturally occurring substances such as Japanese green gentian, clove, the rind of grapes, olive, Panax japonicus C. A. Meyer, carrot and sugar beet while taking into consideration the fact that they are ingested through the oral route. In particular, olive is preferably used and more specifically, preferably used herein are those derived from olive plant and/or products obtained in the olive oil-manufacture processes.

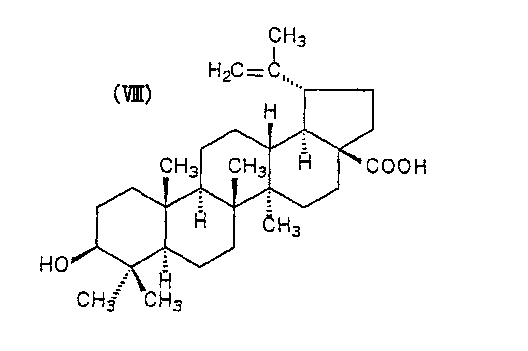

Regarding betulinic acid, physiologically acceptable salts and derivatives thereof are the same as those discussed above.

Examples of physiologically acceptable salts of betulinic acid include, but are not restricted to, sodium betulinate, potassium betulinate, ammonium betulinate, dimethyl-ammonium betulinate, calcium betulinate and magnesium betulinate. Among these, preferred are sodium betulinate and potassium betulinate.

Examples of derivatives of betulinic acid, which are derivatized only at one position, include betulinic acid methyl ester, betulinic acid ethyl ester, betulinic acid n-propyl ester, betulinic acid isopropyl ester, betulinic acid n-butyl ester, betulinic acid trimethylsilyl ester, betulinic acid triethylsilyl ester, betulinic acid β-D-glucopyranosyl ester, betulinic acid β-D-galactopyranosyl ester, 3-O-acetyl-betulinic acid, 3-O- propionyl-betulinic acid, 3-O-butyryl-betulinic acid, 3-O-valeryl-betulinic acid, 3-O- capryl-betulinic acid, 3-O-lauryl-betulinic acid, 3-O-myristyl-betulinic acid, 3-O- palmityl-betulinic acid, 3-O-palmito-oleyl-betulinic acid, 3-O-stearyl-betulinic acid, 3-O-oleyl-betulinic acid, 3-O-vaccenyl-betulinic acid, 3-O-linoleyl-betulinic acid, 3-O-linolenyl-betulinic acid, 3-O-arachidyl-betulinic acid, 3-O-arachidonyl-betulinic acid, 3-O-behenyl-betulinic acid, 3-O-methyl-betulinic acid, 3-O-ethyl-betulinic acid, 3-O-t-butyl-betulinic acid, 3-O-triethylsilyl-betulinic acid, 3-O- β -D-glucopyranosyl-betulinic acid, 3-O- β -D-galactopyranosyl-betulinic acid and 3-O-β-D-glucuronopyranosyl-betulinic acid. Among these, betulinic acid ethyl ester is preferred. The foregoing are derivatives derivatized only at one position or group, but the derivatives usable in the present invention may be those derivatized at two or more possible positions or with two or more groups. Moreover, only derivatives with monosaccharides are listed above as glycosides, but it is a matter of course that the saccharides may be oligosaccharides higher than disaccharides comprising a variety of saccharides.

Betulin is a kind of lupane type triterpenes, has a structure represented by the following chemical formula (IX) and up to this time, it has been known that this compound possesses various effects such as a bio-protein denaturation-inhibitory effect (J.P. KOKAI Hei 9-67253), a glycerol-phosphoric acid dehydrogenase-inhibitory effect (J.P. KOKAI Hei 9-67249), a lipase-inhibitory effect (J.P. KOKAI Hei 10-265328) and a hepatic disease-preventive effect (J.P. KOKAI Hei 11-209275). It has been known that, in nature, betulin is present in, for instance, the bark of white birch. In the foods or beverages and orally administered whitening agent of the present invention, the sources of betulin or derivatives thereof are not restricted to specific ones and may be those derived from natural resources, artificially synthesized ones or commercially available ones, but preferably used herein are those derived from naturally occurring substances such as the bark of white birch while taking into consideration the fact that they are ingested through the oral route.

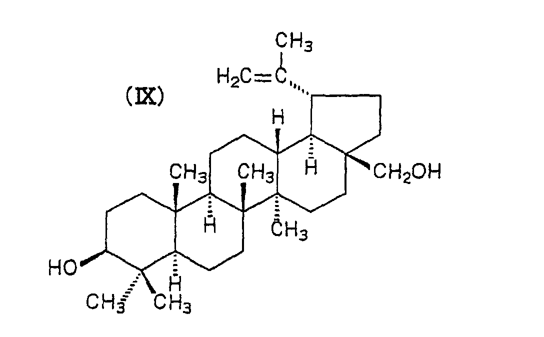

Regarding betulin, physiologically acceptable salts and derivatives thereof are the same as those discussed above.

Examples of derivatives of betulin, which are derivatized only at one position, include 3-O-acetyl-betulin, 3-O-propionyl-betulin, 3-O-butyryl-betulin, 3-O-valeryl-betulin, 3-O-capryl-betulin, 3-O-lauryl-betulin, 3-O-myristyl-betulin, 3-O-palmityl-betulin, 3-O-palmito-oleyl-betulin, 3-O-stearyl-betulin, 3-O-oleyl-betulin, 3-O-vaccenyl- betulin, 3-O-linoleyl-betulin, 3-O-linolenyl-betulin, 3-O-arachidyl-betulin, 3-O-arachidonyl-betulin, 3-O-behenyl-betulin, 28-O-acetyl-betulin, 28-O-propionyl-betulin, 28-O-butyryl-betulin, 28-O-valeryl- betulin, 28-O-capryl-betulin, 28-O-lauryl-betulin, 28-O-myristyl-betulin, 28-O-palmityl-betulin, 28-O-palmito-oleyl-betulin, 28-O-stearyl- betulin, 28-O-oleyl-betulin, 28-O-vaccenyl-betulin, 28-O-linoleyl-betulin, 28-O-linolenyl- betulin, 28-O-arachidyl-betulin, 28-O-arachidonyl-betulin, 28-O-behenyl-betulin, 3-O-methyl-betulin, 3-O-ethyl-betulin, 3-O-t-butyl-betulin, 3-O-triethylsilyl-betulin, 28-O-methyl-betulin, 28-O-ethyl-betulin, 28-O-t-butyl-betulin, 28-O-triethylsilyl-betulin, 3-O- (3 -D-glucopyranosyl-betulin, 3-O- β -D-galactopyranosyl-betulin, 3-O- β -D- glucuronopyranosyl-betulin, 28-O- β -D-glucopyranosyl-betulin, 28-O β -D-galacto- pyranosyl-betulin and 28-O-β-D-glucuronopyranosyl-betulin. Among these, preferred are 3-O-acetyl-betulin and 28-O-acetyl-betulin. The foregoing are derivatives derivatized only at one position or group, but the derivatives usable in the present invention may of course be those derivatized at two or more possible positions or with two or more groups. Examples thereof include 3,28-O-diacetyl-betulin. Moreover, only derivatives with monosaccharides are listed above as glycosides, but it is a matter of course that the saccharides may be oligosaccharides higher than disaccharides comprising a variety of saccharides.

These 5-membered ring-containing triterpenes may be obtained by extracting from naturally occurring substances such as the plant bodies listed above for each compound and more specifically, they may be prepared by extracting the plant body with water and/or an organic solvent and further subjecting the resulting extract to a concentration treatment and/or a fractionation-purification treatment. In other words, each corresponding compound can be obtained by extracting each plant body with water and/or an organic solvent and each corresponding compound can be isolated and/or purified by subjecting the resulting extract to a variety of methods such as solvent-extraction methods, methods, which make use of the difference in solubility between the compound and impurities, fractional precipitation methods, recrystallization methods, ion-exchange resin methods and liquid chromatography techniques, which may be used alone or in any combination or which may repeatedly be used.

In particular, maslinic acid and/or physiologically acceptable salts thereof can be extracted from, for instance, olive plants using water and/or an organic solvent and they can further be isolated and/or purified by subjecting the resulting extract to a variety of methods such as solvent-extraction methods, methods, which make use of the difference in solubility between the compound and impurities, fractional precipitation methods, recrystallization methods, ion-exchange resin methods and liquid chromatography techniques, which may be used alone or in any combination or which may repeatedly be used.

Olive plants (Olea europaea L.) may be used herein irrespective of growing districts (for instance, home-growing or Europe growth ones) or whether they are used for foods or for oil expression. Maslinic acid and/or physiologically acceptable salts thereof to be incorporated into the foods or beverages and orally administered whitening agent of the present invention may be obtained principally from fruits or seeds of olive plant as a natural plant and may likewise be obtained from the rind of the fruits, seed coats, leaves, stems and/or buds or germs of olive plant. Moreover, these compounds can suitably be obtained from dried products, pulverized products and defatted products of the foregoing materials. Among them, preferably used herein are dried and pulverized products of defatted fruits (including rind thereof) and the rinds thereof. Further, these compounds can likewise be obtained from products generated in the olive oil-manufacture processes such as compression residue, extraction residue, oil expression residue, squeezed oil, extracted oil, degummed oil products, deacidified oil products, dark oil, waste decoloring (or whitening) agent, deodorization scum, oil expression juice, waste water and waste filter medium. Among these products, oil expression residue is preferably used in the present invention.

Moreover, the foregoing raw materials such as the fruits of olive plants and the defatted products thereof are preferably humidified by, for instance, the addition of water or by heating with steam, since the raw materials such as the fruits of olive plants and the defatted products thereof get swollen to an appropriate degree and the extraction efficiency thereof is improved.

In particular, the defatted products of olive plants are preferably used in the present invention since they contain maslinic acid and/or physiologically acceptable salts thereof in high concentrations and it is not necessary to remove any oil component from the resulting maslinic acid and/or physiologically acceptable salts thereof.

The defatted products may be obtained from the olive oil expression residue obtained in the edible oil refining steps or the extraction residues obtained after, for instance, extraction with hexane, as raw materials.

In addition, it is also possible to suitably use defatted products obtained by removing the lipid components included in the olive plants or the foregoing defatted products through extraction with at least one solvent selected from the group consisting of hydrocarbons such as pentane, hexane and heptane; lower fatty acid alkyl esters such as acetic acid ethyl ester; and known non-aqueous organic solvents such as diethyl ether and further, if necessary, repeating the foregoing washing treatment.

Thus, maslinic acid and/or physiologically acceptable salts thereof, which can be incorporated into the foods or beverages and orally administered whitening agent of the present invention, can be obtained by extracting the foregoing olive plants with water and/or an organic solvent.

Such organic solvents used when extracting maslinic acid and/or physiologically acceptable salts thereof from olive plants may be either hydrophilic organic solvents or hydrophobic organic solvents. Specific examples thereof include alcohols such as methyl alcohol, ethyl alcohol, glycerin, propylene glycol and 1,3-butylene glycol, and known organic solvents such as acetone, tetrahydrofuran, acetonitrile, 1,4-dioxane, pyridine, dimethylsulfoxide, N,N-dimethylformamide and acetic acid for hydrophilic organic solvents; and known organic solvents such as hexane, cyclohexane, carbon tetrachloride, chloroform, dichloromethane, 1,2-dichloroethane, diethyl ether, ethyl acetate, benzene and toluene for hydrophobic organic solvents. In addition, these organic solvents may be used alone or in any combination of at least two of them.

It is preferred to use hydrophilic organic solvent from the industrial standpoint, for instance, from the viewpoint of the ability of easily penetrating into the plant's tissues and high extraction efficiency. Moreover, water-containing hydrophilic organic solvents are preferably used in the present invention. Specific examples of such organic solvents are alcohols such as methyl alcohol, ethyl alcohol, glycerin, propylene glycol and 1,3-butylene glycol, and known organic solvents such as acetone, tetrahydrofuran and acetonitrile as well as these organic solvents containing water. Maslinic acid and/or physiologically acceptable salts thereof to be incorporated into the foods or beverages and orally administered whitening agent of the present invention can be obtained by extracting olive plants with at least one solvent selected from the group consisting of those listed above.

The extraction conditions are not particularly restricted. For instance, the extraction temperature ranges from 5 to 95°C, preferably 10 to 90°C and more preferably 15 to 85°C and the extraction can suitably be conducted even at ordinary temperature. There would be such a tendency that the extraction efficiency becomes high as the temperature increases. Moreover, the extraction pressure may be ordinary pressure or the extraction can likewise suitably be carried out under a pressure or under a reduced pressure established by, for instance, aspiration. Moreover, the extraction may be conducted according to shaking extraction techniques or using an extraction machine equipped with a stirring machine in order to improve the extraction efficiency. The extraction time may vary depending on other extraction conditions, but in general ranges from several minutes to several hours and the longer the extraction time, the higher the degree of extraction. However, the extraction time may appropriately be determined while taking into consideration the production conditions such as production facilities selected and yield.

In addition, in any case wherein water is used alone, only organic solvent or solvents are used or a mixture of water and at least one organic solvent is used, as an extraction solvent, the amount of the extraction solvent to be used preferably ranges from 1 to 100 times ("mass/mass", those in the following description are shown in the same way also) and more preferably 1 to 20 times the amount of the raw material.

In this connection, the extraction is preferably carried out using, in particular, water, water-containing lower alcohols or anhydrous lower alcohols, while taking into consideration, for instance, the safety to the human bodies.

Furthermore, it is preferred to conduct the extraction using a water-containing lower alcohol having a lower alcohol content of not less than 10% by mass, while taking into consideration the yields of the resulting maslinic acid and/or physiologically acceptable salts thereof and the intensity of the whitening effect thereof. It is more preferred to use a water-containing lower alcohol having a lower alcohol content ranging from 10 to 95% by mass and it is most preferred to use a water-containing lower alcohol having a lower alcohol content ranging from 30 to 95% by mass.

In this respect, examples of alcohols used in the present invention include known solvents, for instance, primary alcohols such as methyl alcohol, ethyl alcohol, 1-propanol and 1-butanol; secondary alcohols such as 2-propanol and 2-butanol; tertiary alcohols such as 2-methyl-2-propanol; and liquid polyhydric alcohols such as ethylene glycol, propylene glycol and 1,3-butylene glycol and these solvents may be used alone or in any combination of at least two of them.

The term "lower alcohol(s)" used herein means known alcohols having 1 to 4 carbon atoms such as the foregoing primary, secondary, tertiary and liquid polyhydric alcohols, which may be used alone or in any combination of at least two of them.

Maslinic acid and/or physiologically acceptable salts thereof used in the present invention can be obtained by the removal of the solvents and moisture from the crude extracted product and/or crude extract thus obtained.

The solvent and moisture can be removed by, for instance, any known technique such as the distillation under reduced pressure, the drying under reduced pressure or in vacuo, the lyophilization and/or the spray drying techniques.

It is a matter of course that the conditions of the crude extracted product and/or crude extract are not particularly restricted and they may be in the states in which they contain solvents and/or moisture.

The extract derived from a defatted product is preferably used herein since it is free of any oil-soluble components such as triglycerides, sterols and tocopherol and thus it is not necessary to remove these oil-soluble components from the extract or to purify the same. Moreover, the term "defatted product" used herein includes residues obtained after oil expression and therefore, the compressed products obtained after the oil expression of olive oil and extraction products may be used. Accordingly, the method is a quite excellent method for effective use of olive plants. In addition, the method uses those, which are commonly abandoned or used as feeds and therefore, it is a quite excellent method even from the viewpoint of the production cost.

Furthermore, it is preferred that maslinic acid and/or physiologically acceptable salts thereof incorporated into the foods or beverages and orally administered whitening agent of the present invention are subjected to, for instance, a concentration treatment in order to further enhance the whitening effect of the masiinic acid and/or physiologically acceptable salts thereof extracted from olive plants.

The conditions for the concentration are not restricted to specific ones and an example thereof is a method, which makes use of the solubility in water. The maslinic acid and/or physiologically acceptable salts thereof incorporated into the foods or beverages and orally administered whitening agent of the present invention have a relatively low polarity and are hardly water-soluble compounds. Therefore, the components hardly soluble and/or insoluble in water (or hardly water-soluble and water-insoluble components) present in the crude extract derived from olive plants can be separated from the easily water-soluble components, while making the most use of the characteristic properties to thus considerably concentrate the extract. The hardly water-soluble and water-insoluble components included in the crude extracted product derived from olive plants have a whitening effect substantially higher than that observed for the entire crude product extracted from olive plants and it can be confirmed that maslinic acid and/or physiologically acceptable salts thereof are concentrated.

The hardly water-soluble and water-insoluble components can easily be obtained by adding the crude product extracted from olive plants to water and stirring the resulting mixture and then collecting the resulting precipitates through, for instance, the filtration.

Moreover, maslinic acid and/or physiologically acceptable salts thereof incorporated into, for instance, the foods or beverages of the present invention can, if necessary, be concentrated by the liquid-liquid partition technique using a general combination of solvents. It is difficult to unconditionally determine such a solvent combination, but examples of such solvent combinations are combinations consisting of water-hydrophobic organic solvents. Examples of such hydrophobic organic solvents include known organic solvents such as hexane, carbon tetrachloride, chloroform, dichloromethane, 1,2-dichloroethane, diethyl ether, ethyl acetate, n-butanol, benzene and toluene, with hexane, ethyl acetate and n-butanol being preferred.

Maslinic acid and/or physiologically acceptable salts thereof are hardly soluble in water and therefore, unnecessary water-soluble components can be removed by separating the hydrophobic organic solvent phase from the aqueous phase. The maslinic acid and/or physiologically acceptable salts thereof can easily be concentrated by the removal of the solvent.

Further, the maslinic acid and/or physiologically acceptable salts thereof to be incorporated into the foods or beverages and orally administered whitening agent of the present invention are preferably fractionated and/or isolated or purified from the foregoing extracted product and/or concentrate. Thus, these substances can be concentrated to an extent higher than that achieved by the foregoing concentration and thus the desired components can be isolated.

Merits of the foregoing fractionation-purification are, for instance, to considerably improve, for instance, the whitening effect of the foregoing substances and to remove impurities. More specifically, the foregoing fractionation-purification treatment is preferred since it permits the achievement of advantages such as the preparation of maslinic acid and/or physiologically acceptable salts thereof in the form of white crystals and the incorporation thereof into, for instance, foods or beverages without being accompanied by any undesirable pigmentation thereof.

In this respect, it is difficult to unconditionally determine such fractionation-purification treatments, but the treatment may be, for instance, the recrystallization technique, the fractional precipitation technique and a technique making use of chromatography. In particular, methods making use of liquid chromatography among others are preferably used in the present invention since they permit the fractionation-purification of maslinic acid and/or physiologically acceptable salts thereof incorporated into the foods or beverages and orally administered whitening agent of the present invention in a high yield without causing any decomposition of these substances. Specific examples of such liquid chromatography techniques usable herein include normal phase liquid chromatography, reverse phase liquid chromatography, thin layer chromatography, paper chromatography and high performance liquid chromatography (HPLC) techniques and either of these methods can be used for the fractionation-purification of maslinic acid and/or physiologically acceptable salts thereof incorporated into the foods or beverages and orally administered whitening agent of the present invention. In particular, preferably used herein are normal phase liquid chromatography, reverse phase liquid chromatography and high performance liquid chromatography (HPLC) techniques while taking into consideration, for instance, the separation efficiency, throughput and step number.

In this connection, the normal phase liquid chromatography is, for instance, the following method. In other words, this method comprises the steps of preparing a column in which the fixed phase comprises, for instance, silica gel and the mobile phase comprises, for instance, a hexane-ethyl acetate mixed liquid or a chloroform-methanol mixed liquid; supplying the crude product extracted from olive plants or the concentrate thereof at a rate of loading ranging from 0.1% to 5% (wt (mass)/v (volume)); and then eluting a desired fraction according to a continuous elution method using a single mobile phase or the stepwise elution method in which the polarity of the solvent is gradually increased.

The "reverse phase liquid chromatography" herein used is, for instance, the following method. In other words, this method comprises the steps of preparing a column in which the fixed phase comprises, for instance, silica coupled with octadecyl silane (ODS) and the mobile phase comprises, for instance, a water-methanol mixed liquid, a water-acetonitrile mixed liquid or a water-acetone mixed liquid; supplying the product extracted from olive plants or the concentrate thereof at a rate of loading ranging from 0.1% to 5% (wt (mass)/v (volume)); and then eluting a desired fraction according to a continuous elution method using a single solvent or the stepwise elution method in which the polarity of the solvent is gradually increased.

The "high performance liquid chromatography (HPLC)" herein used is, in principle, similar to the foregoing normal phase liquid chromatography or the reverse phase liquid chromatography and is a technique for more rapidly carrying out the fractionation-purification at a higher resolution.

The foregoing techniques are preferably used alone or in any combination of at least two of them in the present invention since the use thereof permits the preparation of maslinic acid and/or physiologically acceptable salts thereof in a considerably concentrated and substantially impurity-free condition.

Moreover, the use of the foregoing technique or the use of an appropriate combination of at least two of them would permit the adjustment of the purity of maslinic acid and/or physiologically acceptable salts thereof and the optional design of, for instance, the intensity of the whitening effect and characteristic properties of these substances.

The foregoing concentration treatment can preferably repeatedly be carried out and may further be comprise a combination of different concentration treatments. Similarly, the foregoing fractionation-purification treatment can preferably repeatedly be carried out and may further be comprise a combination of different fractionation-purification treatments. Moreover, it is possible to carry out a fractionation-purification treatment after a concentration treatment; or to carry out a fractionation-purification treatment after a fractionation-purification treatment; or to carry out a concentration treatment, a fractionation-purification treatment and a concentration treatment in this order.

Maslinic acid and/or physiologically acceptable salts thereof can suitably be obtained by variously combining, for instance, the foregoing extraction, concentration and fractionation and/or purification treatments. The combination is not restricted to any specific one, but specific examples of such a series of treatments are as follows.

For instance, olive plants are extracted with water and/or a hydrophilic organic solvent, a part of the whole of the hydrophilic organic solvent is removed from the resulting extract, water is, if needed, added to the resulting product, the mixture is stirred and then the water-insolubles precipitated in the aqueous phase are recovered to thus concentrate the extract. The precipitated water-insolubles may be recovered by, for instance, filtration and/or centrifugation, but the aqueous solution can, if necessary, be subjected to treatments such as addition of water and stirring to improve the efficiency of the recovery. Alternatively, the extract concentrated to dryness obtained by removing the water and/or hydrophilic organic solvent from the extract derived from olive plants may likewise be subjected to treatments such as addition of water and stirring and then the water-insolubles present therein are recovered by, for instance, filtration to thus concentrate the extract. This concentration method is preferred in the present invention since it comprises treatments in an aqueous system, it is excellent in safety as compared with the concentration using an organic solvent and a wide variety of machinery and tools can be used in the method. Moreover, the starting material is almost free of any oil components and therefore, this method is also excellent in the concentration-purification efficiency.

These concentrates can be fractionated and/or purified by the normal phase and/or reverse phase chromatography techniques and/or the recrystallization technique to thus suitably obtain maslinic acid and/or physiologically acceptable salts thereof.

Moreover, the extract obtained from olive plants can be concentrated according to the liquid-liquid partition in a water-hydrophobic organic solvent system and more specifically, the extract can be concentrated by removing the hydrophilic organic solvent from the extract obtained from olive plants, adding, if needed, water to the remaining aqueous solution and then adding a hydrophobic organic solvent. Moreover, the extracted product in the state evaporated to dryness may likewise be concentrated according to the liquid-liquid partition in a water-hydrophobic organic solvent system and more specifically, the extracted product can be concentrated by adding water to the extracted product and then adding a hydrophobic organic solvent. These concentrates can be fractionated and/or purified by the normal phase and/or reverse phase chromatography techniques and/or the recrystallization technique to thus obtain highly purified maslinic acid and/or physiologically acceptable salts thereof.

In this connection, the amount of water to be added upon the liquid-liquid partition is not restricted to any specific one inasmuch as it may permit the desired partition treatment, but the amount preferably ranges from 1 to 100 times, more preferably 5 to 50 times and further preferably about 10 to 30 times the mass of the dried extracted product.

Moreover, in the liquid-liquid partition in a water-hydrophobic organic solvent system, the water-hydrophobic organic solvent system is preferably used in a ratio: water: hydrophobic organic solvent ranging from 9: 1 to 1: 9 (volume ratio) and more preferably 8: 2 to 2: 8.

In addition, the content of the sum of maslinic acid and/or physiologically acceptable salts thereof in the mixture of maslinic acid and/or physiologically acceptable salts thereof obtained from olive plants and/or products generating in the olive oil-manufacture processes is preferably not less than 95% and more preferably 95% to 99.99%. This content can be determined by, for instance, the gas chromatography technique.

The foods or beverages and orally administered whitening agents according to the present invention comprise maslinic acid and/or physiologically acceptable salts thereof, but they can likewise be obtained by the use of the foregoing extracted product and concentrate. In addition, the extent of, for instance, the concentration and/or purification can be controlled to adjust, for instance, the concentration of maslinic acid and/or physiologically acceptable salts thereof and the product having a controlled concentration of the compounds can thus favorably be incorporated into, for instance, foods and beverages. More specifically, if a stronger effect is required, the extract and/or the foregoing extracted product and concentrate can be concentrated, while if they may have a lower effect, a diluted product may be incorporated. In other words, the concentration and conditions of the extract and/or the foregoing extracted product and concentrate may arbitrarily be controlled depending on the purpose of applications.

Further, other whitening substances may be incorporated into these products. This permits the design of the whitening effect of the resulting product in detail and it would be expected that the whitening effect could significantly be enhanced through the synergistic effect of maslinic acid and/or physiologically acceptable salts thereof and other substances having whitening effects. In other words, the whitening effect of the final product can be designed by appropriately adjusting the intensity of the whitening effect and efficacy. The intensity of the whitening effect can be controlled, for instance, by concentration when a higher effect is required or by dilution when a strong effect is not necessary, prior to the incorporation and the whitening effect can thus be controlled to a desired level depending on the purpose of applications. Alternatively, the intensity of the whitening effect can likewise be controlled by combining the whitening components of the present invention such as maslinic acid with whitening components other than the 5-membered ring-containing triterpenes of the present invention. The whitening effect is good for the prevention of sunburn through the screening of UV light rays, the prevention of pigmentation possibly caused after the sunburn and the effect of improving and/or relieving the conditions after the occurrence of such pigmentation. Such efficacy can be controlled by appropriately combining the 5-membered ring- containing triterpenes used in the present invention with whitening components other than the 5-membered ring-containing triterpenes.

Since olive oil contains maslinic acid and therefore, the foods or beverages and orally administered whitening agents of the present invention preferably comprise olive oil as an oil component in order to further improve the whitening effect of the final products.

In addition, when extracting maslinic acid and/or physiologically acceptable salts thereof from olive plants, oleanolic acid and/or physiologically acceptable salts thereof are simultaneously extracted simultaneous with the former. However, the oleanolic acid and/or physiologically acceptable salts thereof are excellent in the compatibility with maslinic acid and therefore, the mixture containing the foregoing components together can directly be incorporated into the foods or beverages of the present invention. In this respect, the use of such a mixture is preferred in the present invention, since it would be expected that these components show synergistic effects based on the physiological effects thereof and, in particular, the use of the mixture would permit the achievement of additive effects with respect to the whitening effect of maslinic acid and/or physiologically acceptable salts thereof. When extracting maslinic acid and/or physiologically acceptable salts thereof from olive plants and isolating and/or purifying the same, these compounds can be obtained as a mixture with oleanolic acid and/or physiologically acceptable salts thereof by appropriately controlling the processing conditions; or maslinic acid and/or physiologically acceptable salts thereof and oleanolic acid and/or physiologically acceptable salts thereof can separately be isolated from olive plants and then the isolated products can be mixed together to give an intended mixture of these compounds. Alternatively, maslinic acid and/or physiologically acceptable salts thereof and oleanolic acid and/or physiologically acceptable salts thereof can be isolated from different raw materials and then the isolated products can be mixed together to give an intended mixture of these compounds.

Incidentally, the 5-membered ring-containing triterpenes other than maslinic acid and/or physiologically acceptable salts thereof can likewise be isolated from natural resources in accordance with materials and methods explained above in connection with maslinic acid and/or physiologically acceptable salts thereof.

The foods, beverages or orally administered whitening agents are preferably prepared using products isolated from natural substances since they are not influenced by the impurities originated from the natural substances and they are in colorless to slightly colored and/or odorless to almost odorless conditions. Accordingly, if 5-membered ring-containing triterpenes and physiologically acceptable salts or derivatives thereof are isolated from natural substances and they are used for the preparation of cuisine, they never adversely affect the taste and texture thereof. In particular, they can be incorporated into cuisine, which does not require any taste and texture of a natural substance as a raw material such as olive. Accordingly, the foods and beverages of the present invention include those capable of cooking or incorporation of such ingredients irrespective of the kinds of natural substances used as raw materials.

Moreover, when olive and/or olive oil are ingested as such without any pre-treatment, one can ingest only a small amount of the desired 5-membered ring- containing triterpenes of the present invention, but if ingesting a food or beverage or an orally administered whitening agent comprising the 5-membered ring-containing triterpenes isolated from natural substances and incorporated into the same, one can relatively easily ingest a large amount of the 5-membered ring-containing triterpenes.

Incidentally, the 5-membered ring-containing triterpenes included in, for instance, olive are oil-soluble substances on the whole, in general exist in oil and fats and for this reason, it is difficult to incorporate these components into an aqueous food or beverage, but the 5-membered ring-containing triterpenes isolated from natural substances can be incorporated into either oil-based foods or beverages or aqueous foods or beverages. If these compounds are converted into an aqueous food or beverage such as a refreshing drink, one can easily ingest the intended 5-membered ring-containing triterpenes of the present invention in an amount, for instance, ranging from several grams to several tens of grams.

Moreover, the food or beverage or orally administered whitening agent of the present invention containing the 5-membered ring-containing triterpenes isolated from natural substances is completely free of any impurity or contaminate capable of inhibiting the whitening effect and the prevention of the internal absorption of these compounds and therefore, a favorable whitening effect of the skin can be achieved.

Moreover, the 5-membered ring-containing triterpenes incorporated into the foods or beverages and orally administered whitening agents of the present invention have a melanin production-inhibitory function.

The term "melanin production-inhibitory function" used herein means the function of inhibiting biosynthesis of melanin pigment by melanocytes stimulated by, for instance, the irradiation with UV light rays, hormone abnormality and genetic information. In general, it has been recognized that the dark skin, liver spots, ephelis and dark area of the skin are generated when melanocytes are stimulated by, for instance, the irradiation with UV light rays and the hormone abnormality and the melanin pigment synthesized therein is precipitated in the skin. Accordingly, if the melanin production can be inhibited, it would be possible to prevent or relieve the dark skin, liver spots, ephelis and dark area of the skin. In other words, the foods or beverages and orally administered whitening agents of the present invention may have a melanin production-inhibitory function by incorporating 5-membered ring-containing triterpenes into these products. Thus, the products can internally show the function of the 5-membered ring-containing triterpenes when ingesting the same and as a result, the product would contribute to the maintenance of white and beautiful skin.

The melanin production-inhibitory function of the 5-membered ring-containing triterpenes incorporated into the foods or beverages and orally administered whitening agents of the present invention can be determined by a test method, which makes use of cultured melanocytes. The term "melanocyte(s)" used herein means cells having an ability of producing melanin and when cultivating these cells in the usual manner, the cells are blackened due to the accumulation of melanin pigment thus produced. Contrary to this, if a substance having such a melanin production-inhibitory function is incorporated into this cultivation system, the melanin production is suppressed and the skin is relatively whitened. The melanin production-inhibitory function can likewise be evaluated on the basis of this degree of relative whitening.

According to the evaluation by the foregoing test method, the whitening effects of the 5-membered ring-containing triterpenes and physiologically acceptable salts or derivatives thereof incorporated into the foods or beverages and orally administered whitening agents of the present invention are very high as compared with that observed for magnesium ascorbate phosphate as a known orally administered whitening agent. More specifically, the whitening effect (as expressed in terms of the melanin production-inhibitory function) ranges from about 100 to 200 times that observed for the known orally administered whitening agent in case of maslinic acid, about 100 to 200 times for maslinic acid salts, about 10 to 100 times for erythrodiol, about 100 to 200 times for ursolic acid, about 30 to 120 times for uvaol, about 30 to 120 times for betulinic acid, about 10 to 100 times for betulin, about 100 to 200 times for maslinic acid ethyl ester, about 50 to 150 times for acetylated maslinic acid, about 30 to 120 times for triethyl-silylated maslinic acid, about 50 to 150 times for stearoyl-maslinic acid ethyl ester, about 10 to 100 times for acetylated erythrodiol, about 100 to 200 times for ursolic acid ethyl ester, about 10 to 100 times for acetylated uvaol, about 10 to 100 times for betulinic acid ethyl ester and about 5 to 50 times for acetylated betulin. In other words, the foods or beverages and orally administered whitening agents of the present invention contain the 5-membered ring-containing triterpenes and physiologically acceptable salts or derivatives thereof incorporated therein and therefore, they can enjoy quite strong melanin production-inhibitory effects of these compounds.

The 5-membered ring-containing triterpenes, and alcohol ester group-containing derivatives, fatty acid ester group-containing derivatives, alkoxy group-containing derivatives and alkoxymethyl group-containing derivatives thereof are, as a whole, oil-soluble and therefore, they can suitably be incorporated into oil-based or emulsion type foods or beverages and orally administered whitening agents. In addition, these compounds are preferably ingested in the form of oil and fats preparations or processed oil and fats from the viewpoint of the absorptive properties, since they are expected to be absorbed together with the oil component.

Moreover, the physiologically acceptable salts or glycosides of 5-membered ring-containing triterpenes are, on the whole, water-soluble and therefore, they can be used in, for instance, aqueous or emulsion type foods or beverages and orally administered whitening agents by incorporating these compounds into the same in uniformly dissolved or dispersed states. In particular, beverages or the like are frequently sold in the form of aqueous or emulsified conditions and therefore, the 5-membered ring-containing triterpenes can favorably be incorporated into these products in the form of physiologically acceptable salts or glycosides thereof.

Moreover, the 5-membered ring-containing triterpenes and/or physiologically acceptable salts thereof permit the achievement of the currently required whitening effect by the incorporation of these compounds into foods or beverages and orally administered whitening agents in quite small amounts and therefore, the use thereof is advantageous in the production cost. In addition, there may be much room for the incorporation of other components from the viewpoint of the compounding ratio and therefore, other functions of these products can further be enriched.

Thus, according to the present invention, foods or beverages and orally administered whitening agents having, for instance, considerably excellent whitening effect can of course be prepared by increasing the amount of the 5-membered ring-containing triterpenes and/or physiologically acceptable salts thereof to be incorporated into the same.

The foods or beverages and orally administered whitening agents according to the present invention can show their whitening effect by the oral administration of the same.

The term "whitening effect" herein used means an effect of preventing or relieving the dark skin, liver spots, ephelis and dark area of the skin generated due to a variety of factors such as irradiation with UV light rays, a change in the hormone balance and genetic program; an effect of making the skin transparent and beautiful or an effect of maintaining transparent and beautiful skin; and an effect of relieving the dullness of the skin and increasing the gloss and tenseness of the skin. In general, it has been recognized that the dark skin, liver spots, ephelis and dark area of the skin are generated when melanocytes are stimulated by a variety of factors such as the stimulation by UV light rays and a change in the hormone balance and the melanin pigment biosynthesized in the cells are precipitated in the skin. Therefore, for instance, the dark skin, liver spots, ephelis, dark area and dullness of the skin can be prevented or relieved if the melanin production can be inhibited. In this respect, the foods or beverages and orally administered whitening agents of the present invention are quite preferred since they comprise the 5-membered ring-containing triterpenes having a melanin production-inhibitory function and they may thus suppress the melanin production to a level as low as possible. In other words, the foods or beverages and orally administered whitening agents of the present invention can show their whitening effect by the oral administration of the same and accordingly, they can substantially contribute to the relief or prevention of the dark skin, liver spots, ephelis, dark area and dullness of the skin and the maintenance of transparent and beautiful skin.

The whitening effect of the foods and beverages can, for instance, be evaluated by allowing animals to ingest feeds containing 5-membered ring-containing triterpenes and determining the degree of improvement of the skin pigmentation, which may be used as an indication of the whitening effect.

More specifically, the whitening effect can be evaluated by removing a half of the hair on the back of each brown guinea pig, keeping these animals over 2 weeks while irradiating the hair-removed area with a UV lamp, dividing these guinea pigs into groups in such a manner that there is not any difference in the degree of the melanin pigmentation induced by the UN light irradiation, allowing the animals in each group to freely take a test food comprising a feed having a low vitamin C content to which a variety of triterpenes have been added and keeping these animals over additional 4 weeks to evaluate the degree of skin pigmentation while comparing the same with those observed for a control group and for a positive control group.

The results obtained in such evaluation clearly indicate that the oral administration of the 5-membered ring-containing triterpenes would permit relief of the skin pigmentation in its early stage or they can make the skin pigmentation inconspicuous.

The whitening effect of foods and beverages in man can be evaluated by, for instance, preparing tablet-like sweet (confectionery) containing 5-membered ring-containing triterpenes, allowing the panel to practically take the confectionery to thus evaluate the dark skin, liver spots, ephelis, dark area and dullness of the skin using the degree of relief of the same as an indication.

More specifically, 25 to 50-year-old 30 women are randomly divided into two sections (15 women each) or a control section and a test section, allowing the women in the test section to taste the tablet-like sweet containing 5-membered ring-containing triterpenes, while allowing the women in the control section to taste similar tablet-like sweet free of any triterpene, simultaneous with the meals (breakfast, lunch and supper), requesting each panelist to self-evaluate the degree of relief in the dark skin, liver spots, ephelis, dark area and dullness of the skin after 12 weeks to thus evaluate the whitening effect of the foods and beverages.

The results obtained in such evaluation clearly indicate that the foods or beverages and orally administered whitening agents of the present invention permit the prevention of the skin pigmentation or the dark skin, liver spots, ephelis, dark area and dullness of the skin and make the skin pigmentation inconspicuous and make the skin beautiful, as compared with similar foods or beverages free of any 5-membered ring- containing triterpene.

Moreover, the present invention relates to a food or beverage comprising at least one member selected from the group consisting of 5-membered ring-containing triterpenes and physiologically acceptable salts or derivatives thereof and in particular, to a food or beverage comprising, for instance, the 5-membered ring-containing triterpenes as whitening components. The passage "comprising as whitening components" herein used means that the food or beverage comprises the components in an amount sufficient for achieving the desired whitening effect and that the components are incorporated into the food or beverage in expectation of achieving a desired whitening effect.

In the foods or beverages of the present invention, the content of the 5-membered ring-containing triterpenes and physiologically acceptable salts or derivatives thereof is not restricted to any specific range and as has been described above, the content thereof may appropriately be adjusted depending on a variety of factors such as the kinds of 5-membered ring-containing triterpenes selected, the kinds of foods or beverages, the amount of the foods or beverages to be ingested, the frequency of the ingestion and the body weight and sex of a person who ingests the foods or beverages, but the content is, for instance, in the range of from 0.00001 to 50% by mass, preferably 0.0001 to 30% by mass, more preferably 0.001 to 20% by mass, particularly preferably 0.01 to 15% by mass, further preferably 0.1 to 10% by mass, further preferably 0.5 to 10% by mass, further preferably 1 to 10% by mass and most preferably 2 to 10% by mass. The same is true for the case wherein the compounds are used as whitening components and the content thereof is not particularly restricted.

The food or beverage of the present invention comprises, for instance, the foregoing 5-membered ring-containing triterpenes and therefore, it possesses a variety of effects originated from the compounds and it is characterized, in particular, in that it has an effect of whitening the skin. The product of the present invention is a food or beverage and therefore, it can easily and continuously be ingested and it would be expected that the food or beverage shows an excellent effect.

Furthermore, the present invention also relates to the foregoing food or beverage wherein the foregoing 5-membered ring-containing triterpene is preferably at least one member selected from the group consisting of oleanane type triterpenes represented by the general formula (I), ursane type triterpenes represented by the general formula (II) and lupane type triterpenes represented by the general formula (III). They are preferred because of their particularly strong whitening effects.

Among the foregoing oleanane type triterpenes, preferred are maslinic acid and erythrodiol, with maslinic acid being particularly preferred. Among the foregoing ursane type triterpenes, preferably used herein are ursolic acid and uvaol. Among the foregoing lupane type triterpenes, preferably used herein are betulinic acid and betulin. The content of these compounds is not particularly restricted as has been described above, but it ranges, for instance, from 0.00001 to 50% by mass, preferably 0.0001 to 30% by mass, more preferably 0.001 to 20% by mass, particularly preferably 0.01 to 15% by mass, further preferably 0.1 to 10% by mass, further preferably 0.5 to 10% by mass, further preferably 1 to 10% by mass and most preferably 2 to 10% by mass. The same is true for the case wherein the compounds are used as whitening components and the content thereof is not particularly restricted.

Moreover, the food or beverage of the present invention may comprise, for instance, other physiologically active components in order to, for instance, improve the functions of the food or beverage, in particular, synergistically improve the whitening effect thereof, make up for the whitening effect and improve the absorbing power or capacity. Examples of such other physiologically active components include, but are not restricted to, other orally administered whitening agents, which may have synergistic whitening effects, anti-oxidant components, which may indirectly contribute to the whitening effect, oily components, which can improve the internal absorptivity and which can, in turn, improve the efficacy of the whitening components and various kinds of vitamins, minerals and amino acids for the nutritional enrichment.

Examples of other orally administered whitening agents are vitamin C and derivatives and salts thereof, kojic acid and derivatives thereof, hydroquinone and derivatives thereof, pro-anthocyanidins, iso-flavonoid and derivatives thereof, tannins, SH group containing preparations such as cysteine and glutathione, colloidal sulfur, peptides having specific amino acid sequences, placenta extract, strawberry geranium extract, coix seed extract, Scutellaria baicalensis Georg. extract, marine algae extract and wheat extract. Among these orally administered whitening agents, particularly preferred are vitamin C and derivatives and salts thereof, pro-anthocyanidins, iso-flavonoid and derivatives thereof and tannins. It would be expected that these orally administered whitening agents show synergistic effects with the 5-membered ring-containing triterpenes of the present invention and thus they can preferably be used in the present invention. When simultaneously using these whitening agent in the food or beverage of the present invention, the whitening agent is used in a ratio (mass ratio relative to the food or beverage of the present invention) preferably ranges from 0: 100 to 99.99: 0.01. When using placenta extract and plant extracts without any pre-treatment, it is sufficient that the amount thereof as expressed in terms of the dry solid content falls within the range specified above and they show excellent whitening effects if the contents thereof fall within the range.

The foregoing antioxidant components are not restricted to specific ones inasmuch as they have currently been used in foods and beverages, but specific examples thereof are vitamin C and derivatives and salts thereof; tocopherol and tocotrienol and derivatives thereof; dibutyl hydroxy toluene, butyl hydroxy anisole, disodium ethylenediaminetetraacetate, calcium disodium ethylenediaminetetraacetate; tannins of gallic acid and ellagic acid and derivatives thereof; sulfuric acid type compounds such as sodium sulfite, sodium hyposulfite and sulfur disulfide; ferulic acid derivatives such as γ -oryzanol; rutin and derivatives thereof; lignans and glycosides thereof such as sesamin, epi-sesamin, sesaminol, sesamolin and sesamol; carotenoids and derivatives thereof such as β carotene; flavonoids such as flavone, catechin, quercetin, iso-quercetin, leuco-anthocyanidin, genistin, genistein, 6"-O-acetylgenistin, 6"-O-malonyl genistein, daidzin, daidzein, 6"-O-acetyldaidzin, 6"-O-malonyl daidzin, glycitin, glycitein, 6"-O-acetylglycitin, 6"-O-malonyl glynitin, puerarin, quercetin, kaempferol and miroestrol; quinones such as ubiquinone and vitamin K; enzymes such as superoxide dismutase, catalase and glutathione peroxidase; mallow flower extract, Aspergillus terreus extract, licorice oil-extract, clove extract, guaiac resin, green coffee bean extract, rice bran oil extract, canna extract, sage extract, dropwort (Japanese parsley) extract, tempeh extract, rape seed oil extract, pimenta extract, blue berry extract, propolis extract, pepper extract, melaleuca extract, eucalyptus extract, gentiana extract, buckwheat extract, adzuki bean extract and rosemary extract; oil cake extracts such as olive oil cake extract and soybean oil cake extract; soybean germ extract; thiamines and salts thereof; riboflavins such as riboflavin and riboflavin acetate; pyridoxines such as pyridoxine hydrochloride and pyridoxine dioctanoate; nicotinic acids such as nicotinic acid amide and benzyl nicotinate; bilirubin, mannitol, tryptophane, histidine and nordihydro-guaiaretic acid. The use of these antioxidants is preferred in the present invention since it has been recognized that these antioxidants indirectly show whitening effects and it would be expected that they can provide overall synergistic effects on the skin through the antioxidant effects peculiar thereto such as a blood circulation-promoting effect and an anti-aging effect.

The oil components are not particularly restricted and include, for instance, vegetable oils such as soybean oil, rape seed oil, sesame oil and olive oil; animal oils such as lard, beef tallow and fish oil; MCT, MLCT, diglycerides and monoglycerides such as those isolated from natural resources and those synthesized by chemical reactions and enzyme reactions; and structural oil and fats in which the structures of the fatty acids are variously designed, but the present invention is not restricted to these specific examples.

Further, additives for nutritional enrichment such as various kinds of vitamins, minerals and amino acids are not restricted to specific ones and it is desirable to use those specified in Japanese Standards of Food Additives.

Furthermore, the food or beverage of the present invention may comprise various raw materials commonly used in the usual foods and beverages. Examples of such raw materials usable herein include, but are not limited to, a variety of seasonings such as miso, soy sauce, sauce, ketchup, bouillon, sauce for roast meat, roux for curry, premix for stew, premix for soup and premix for soup stock; animal oil and fats such as lard, beef tallow and milk fat; oil and fats derived from marine products such as whale oil, sardine oil and herring oil; vegetable oils such as soybean oil, rape seed oil, cotton seed oil, rice bran oil, corn oil, sesame oil, peanut oil, sunflower oil, safflower oil, camellia oil, olive oil, linseed oil, tung oil, castor oil, coconut oil, palm oil and cacao butter; thickening agents such as xanthane gum; sweeteners such as sugar, granulated sugar, lactose, fructose, dextrose, sorbitol and honey; savory seasonings such as MSG (monosodium glutamine); edible vinegars such as rice vinegar, cider vinegar and vinegar derived from alcohol; acidulants such as citric acid, malic acid, lactic acid and tartaric acid; synthetic preservatives such as sodium benzoate; common salt, pepper and flavors. In particular, olive oil is quite preferred in the present invention because it comprises, for instance, maslinic acid used in the present invention.

The foregoing components can appropriately be designed and added to the food or beverage of the present invention depending on the purpose of applications. It is thus possible to synergistically improve and/or compensate the whitening effect depending on the absorptivity and kinds of functions and/or effects and to make the manner of use preferable. Moreover, substances such as iso-flavones and derivatives thereof are excellent in water-solubility and if acting these substances on living bodies together with the 5-membered ring-containing triterpenes of the present invention, which are on the whole oil-soluble substances, they show a variety of effects including tyrosinase- inhibitory effect and simultaneously acting effects through a variety of water and lipid-mediated metabolic pathways and it would be expected that these effects are synergistic ones. Moreover, in products such as foods and beverages for whitening, which comprise both the 5-membered ring-containing triterpenes and other substances such as iso-flavonoid, it would be expected that the physiological activities of iso-flavonoid such as antioxidant properties and estrogen-like functions are simultaneously and synergistically activated.

Specific examples of the foods and beverages of the present invention will be listed below, but the present invention is not restricted to these specific examples at all. The food and beverage of the present invention are not restricted in, for instance, their shapes and may be in the form of, for instance, the usual shapes, liquid foods, nutritional food ingested through intestines, health foods and foods for babies and little children. Specific examples of such foods and beverages are Japanese-style confections such as OKAKI, rice crackers, millet-and-rice cakes, buns with bean-jam filling and wheat gluten; various kinds of confectionery such as cookies, biscuits, crackers, cereal foods, pie, castilla, doughnuts, custard pudding, sponge cakes, waffle, butter cream, custard cream, cream puff (choux a la crème), chocolate, chocolate confectionery, caramel, candy, chewing gum, jelly, hot cake, bread and buns; snack confectionery such as potato chips; frozen deserts such as ice cream, ice candy and sherbet; refreshing drinks such as sour milk beverages, lactic acid bacteria-containing beverages, thick lactescent beverages, fruit juice drinks, flesh-containing drinks (so-called nectar), functional drinks and carbonated drinks; table luxuries and beverages thereof such as green tea, black tea, coffee and cocoa; alcoholic drinks such as sake, wine, brandy, whisky, alcoholic drinks for medical use; dairy products such as cow's milk, fermented milk, processed milk and cheese; processed soybean foods such as soybean milk and soybean curd (tofu); jam, fruits dipped in syrup; pastes such as flower paste, peanut paste and fruit paste; pickles (salted vegetables or the like), cereal products such as wheat vermicelli and pasta; meat products such as ham, sausage, bacon, dry sausage, beef jerky and hamburg; fishery products such as fish meat ham, fish meat sausage, boiled fish paste (kamaboko), fish stick (chikuwa) and floated-type kamaboko; dried fishes such as those of fishes and shellfishes; dried fishes such as dried bonito, mackerel and horse mackerel; salted fish guts such as those of sea urchins and cuttlefishes; dried mirin-seasoned products of dried cuttlefishes and fishes; smoked products such as smoked salmon meat; foods boiled down in soy sauce such as laver, small fishes, shellfishes, edible wild plants, shiitake and sea tangles; retort foods such as curry and stew; a variety of seasonings such as miso, soy sauce, sauce, ketchup, bouillon, sauce for roast meat, roux for curry, premix for stew, premix for soup and premix for soup stock; cooked rice products; processed oil and fats such as oil and fats preparations, margarine, shortening, mayonnaise and dressings; and a variety of products for cooking in a range and frozen foods, which contain oil and fats preparations. Among these foods and beverages, particularly preferred are cooked rice, a variety of seasonings, processed oil and fats such as oil and fats preparations, margarine, shortening, mayonnaise and dressings from the viewpoint of continuous ingestion. Moreover, the foods and beverages are not limited in their shapes and properties and may be in any shapes such as solid, semi-solid, gel-like, liquid-like and powdery shapes.

The 5-membered ring-containing triterpenes used in the food or beverage of the present invention are, by nature, oil-soluble substances and therefore, the food or beverage of the present invention is preferably oil and fats and foods obtained by processing oil and fats from the viewpoint of the solubility thereof. Such oil and fats are not restricted to specific ones, but they may, for instance, be oil and fat preparations obtained by incorporating naturally occurring or artificially synthesized 5-membered ring-containing triterpenes into the usual oil and fats through dissolution; oil and fat preparations obtained by appropriately adjusting the conditions for compressing and extracting plant's seeds so that the 5-membered ring-containing triterpenes present in the seeds are contained in the compressed and/or extracted oils; and oil and fat preparations obtained by adjusting the conditions for the purification in such a manner that the 5-membered ring-containing triterpenes present in the oils remain therein. In addition, it is also possible to admix an oil and fat preparation having a high content of the 5-membered ring-containing triterpenes with other oil and fats and the achievement of synergistic effects with the physiological effects of the trace components included in the other oil and fats would be expected in this case.

The 5-membered ring-containing triterpenes can likewise be obtained from plants as raw materials for oils and fats and therefore, oil and fat preparations are preferred from the viewpoint of the production. Further, preferably used herein also include products obtained by processing oils and fats such as margarine, shortening, mayonnaise and dressings, which are products obtained by processing oil and fat preparations.

Similarly, products obtained by the use of, for instance, the foregoing oil and fat preparations of the present invention are likewise preferred. In this respect, the term "use" means the use as a raw material and the use in fried foods and roasted foods or the use as so-called oil and fat preparations.

In this respect, the 5-membered ring-containing triterpenes can be used in foods and beverages without any restriction, but it is preferred, in case of oil-based foods and beverages, to use 5-membered ring-containing triterpenes, alcohol ester group-containing derivative, fatty acid ester group-containing derivatives, alkoxy group- containing derivatives and alkoxymethyl group-containing derivatives thereof. These compounds are relatively highly oil-soluble and accordingly, they can be applied to oil-based foods and beverages. Moreover, physiologically acceptable salts or glycosides of 5-membered ring-containing triterpenes can of course be incorporated into these foods and beverages, but it is preferred to use an emulsifying agent in this case.

Moreover, physiologically acceptable salts or glycosides of 5-membered ring- containing triterpenes are in general preferably used in water-based foods and beverages. They have relatively high solubility in water and therefore, they can suitably be used in water-based foods and beverages. Moreover, other 5-membered ring-containing triterpenes or derivatives thereof can of course be incorporated into these foods and beverages, but it is preferred to use an emulsifying agent in this case.

When ingesting the food or beverage of the present invention, the 5-membered ring-containing triterpenes and physiologically acceptable salts or derivatives thereof having skin-whitening effects are internally absorbed and one can thus enjoy the whitening effects. The product of the present invention is in the form of a food or beverage and therefore, it can continuously be ingested unlike the cosmetics, which require a great deal of labor.

The present invention relates to an orally administered whitening agent comprising, as an effective component, at least one member selected from the group consisting of 5-membered ring-containing triterpenes and physiologically acceptable salts or derivatives thereof.

The present invention also relates to the foregoing orally administered whitening agent in which the 5-membered ring-containing triterpene is at least one member selected from the group consisting of oleanane type triterpenes represented by the general formula (I), ursane type triterpenes represented by the general formula (II) and lupane type triterpenes represented by the general formula (III). Among the foregoing oleanane type triterpenes, preferred are maslinic acid and erythrodiol, with maslinic acid being particularly preferred. Among the foregoing ursane type triterpenes, preferred are ursolic acid and uvaol. Among the foregoing lupane type triterpenes, preferred are betulinic acid and betulin.

The orally administered whitening agent of the present invention possesses melanin production-inhibitory effect and therefore, it is used as a prophylactic and/or therapeutic agent for, in particular, the dark skin, liver spots, ephelis, dark area and dullness of the skin. The term "use as a prophylactic agent" means the use of the whitening agent for suppressing the generation of the dark skin, liver spots, ephelis, dark area and dullness of the skin through the inhibition of the melanin production. The term "use as a therapeutic agent" means the use of the whitening agent for the elimination of the dark skin, liver spots, ephelis, dark area and dullness of the skin or for making these symptoms inconspicuous by more strongly suppressing the melanin production or by the immediate decomposition of the melanin produced to thus reduce the overall amount of melanin.

The orally administered whitening agent of the present invention can be prepared by forming such 5-membered ring-containing triterpenes and physiologically acceptable salts or derivatives thereof into, for instance, solid pharmaceutical preparations such as tablets, powders, capsules, granules and pills; or liquid preparations such as aqueous solutions, suspensions and emulsions. Specific examples thereof include, but are not limited to, tablets such as uncoated tablets, sugar-coated tablets, coating tablets, enteric coated tablets, chewable tablets, buccal tablets, sublingual tablets, troche and adhesive tablets; powders; capsules such as hard capsules and soft capsules; coated products, pills, troche, liquid preparations, or granules such as pharmaceutically acceptable sustained release preparations thereof, aqueous solutions for internal use, shake mixtures, suspensions, emulsions, syrups, dry syrups, elixir, infusion, decoction, and liquid preparations such as limonade. These pharmaceutical preparations can be prepared by adding, to the foregoing effective components, additives commonly used in the orally administered agent such as excipients, binders, disintegrators, lubricants, coating agents, bases, suspending agents, emulsifying agents, humectants, preservatives, stabilizers, surfactants and corrigents and forming the resulting mixture into a desired shape according to the usual method. Among the foregoing pharmaceutical preparations, preferred are uncoated tablets, sugar-coated tablets, coating tablets, enteric coated tablets, chewable tablets, powders, capsules and granules.

In addition, the orally administered whitening agent of the present invention may simultaneously comprise other physiologically active components for the purpose of, for instance, the synergistic improvement of the whitening effect, the compensation of the whitening effect and the improvement of the absorptivity. Such other physiologically active components are not particularly restricted, but examples thereof include other orally administered whitening agents, which may have synergistic whitening effects, anti-oxidant components, which may indirectly contribute to the whitening effect, oily components, which can improve the internal absorptivity and which can, in turn, improve the efficacy of the whitening components and various kinds of vitamins, minerals and amino acids for the nutritional enrichment.

Examples of other orally administered whitening agents are vitamin C and derivatives and salts thereof, kojic acid and derivatives thereof, hydroquinone and derivatives thereof, pro-anthocyanidins, iso-flavonoid and derivatives thereof, tannins, SH group containing preparations such as cysteine and glutathione, colloidal sulfur, peptides having specific amino acid sequences, placenta extract, strawberry geranium extract, coix seed extract, Scutellaria baicalensis Georg. extract, marine algae extract and wheat extract. Among these orally administered whitening agents, particularly preferred are vitamin C and derivatives and salts thereof, pro-anthocyanidins, iso-flavonoid and derivatives thereof and tannins. It would be expected that these orally administered whitening agents show synergistic effects with the 5-membered ring-containing triterpenes of the present invention and thus they can preferably be used in the present invention. When simultaneously using these whitening agent in the orally administered whitening agent of the present invention, the whitening agent is used in a ratio (mass ratio relative to the orally administered whitening agent of the present invention) preferably ranges from 0: 100 to 99.99: 0.01. When using placenta extract and plant extracts without any pre-treatment, it is sufficient that the amount thereof as expressed in terms of the dry solid content falls within the range specified above and they show excellent whitening effects if the contents thereof fall within the range.

The foregoing antioxidant components are not restricted to specific ones inasmuch as they have currently been used in foods and beverages, but specific examples thereof are vitamin C and derivatives and salts thereof; tocopherol and tocotrienol and derivatives thereof; dibutyl hydroxy toluene, butyl hydroxy anisole, disodium ethylenediaminetetraacetate, calcium disodium ethylenediaminetetraacetate; tannins of gallic acid and ellagic acid and derivatives thereof; sulfuric acid type compounds such as sodium sulfite, sodium hyposulfite and sulfur disulfide; ferulic acid derivatives such as γ -oryzanol; rutin and derivatives thereof; lignans and glycosides thereof such as sesamin, epi-sesamin, sesaminol, sesamolin and sesamol; carotenoids and derivatives thereof such as β carotene; flavonoids such as flavone, catechin, quercetin, iso-quercetin, leuco-anthocyanidin, genistin, genistein, 6"-O-acetylgenistin, 6"-O-malonyl genistein, daidzin, daidzein, 6"-O-acetyldaidzin, 6"-O-malonyl daidzin, glycitin, glycitein, 6"-O-acetylglycitin, 6"-O-malonyl glynitin, puerarin, quercetin, kaempferol and miroestrol; quinones such as ubiquinone and vitamin K; enzymes such as superoxide dismutase, catalase and glutathione peroxidase; mallow flower extract, Aspergillus terreus extract, licorice oil-extract, clove extract, guaiac resin, green coffee bean extract, rice bran oil extract, canna extract, sage extract, dropwort (Japanese parsley) extract, tempeh extract, rape seed oil extract, pimenta extract, blue berry extract, propolis extract, pepper extract, melaleuca extract, eucalyptus extract, gentiana extract, buckwheat extract, adzuki bean extract and rosemary extract; oil cake extracts such as olive oil cake extract and soybean oil cake extract; soybean germ extract; thiamines and salts thereof; riboflavins such as riboflavin and riboflavin acetate; pyridoxines such as pyridoxine hydrochloride and pyridoxine dioctanoate; nicotinic acids such as nicotinic acid amide and benzyl nicotinate; bilirubin, mannitol, tryptophane, histidine and nordihydro-guaiaretic acid. The use of these antioxidants is preferred in the present invention since it has been recognized that these antioxidants indirectly show whitening effects and it would be expected that they can provide overall synergistic effects on the skin through the antioxidant effects peculiar thereto such as a blood circulation-promoting effect and an anti-aging effect.

The oil components are not particularly restricted and include, for instance, vegetable oils such as soybean oil, rape seed oil, sesame oil and olive oil; animal oils such as lard, beef tallow and fish oil; MCT, MLCT, diglycerides and monoglycerides such as those isolated from natural resources and those synthesized by chemical reactions and enzyme reactions; and structural oil and fats in which the structures of the fatty acids are variously designed, but the present invention is not restricted to these specific examples.

Further, additives for nutritional enrichment such as various kinds of vitamins, minerals and amino acids are not restricted to specific ones and it is desirable to use those specified in Japanese Standards of Food Additives.

The foregoing components can appropriately be designed and added to the orally administered whitening agent of the present invention depending on the purpose of applications. It is thus possible to synergistically improve and/or compensate the whitening effect depending on the absorptivity and kinds of functions and/or effects and to make the manner of use preferable. Moreover, substances such as iso-flavones and derivatives thereof are excellent in water-solubility and if acting these substances on living bodies together with the 5-membered ring-containing triterpenes of the present invention, which are on the whole oil-soluble substances, they show a variety of effects including tyrosinase-inhibitory effect and simultaneously acting effects through a variety of water and lipid-mediated metabolic pathways and it would be expected that these effects are synergistic ones. Moreover, in products such as orally administered whitening agents, which comprise both the 5-membered ring-containing triterpenes and other substances such as iso-flavonoid, it would be expected that the physiological activities of iso-flavonoid such as antioxidant properties and estrogen-like functions are simultaneously and synergistically activated.

The orally administered whitening agent of the present invention possesses the whitening effect as has been discussed above. More specifically, one can enjoy the whitening effect of the whitening agent by directly or indirectly ingesting the orally administered whitening agent of the present invention. Moreover, if continuously ingesting the whitening agent, one can enjoy more excellent effect. The term "comprising as an effective component" means that the agent comprises the foregoing compounds in an amount sufficient for achieving the desired whitening effect. The amount of the 5-membered ring-containing triterpenes and physiologically acceptable salts or derivatives thereof to be incorporated into the orally administered whitening agent of the present invention is not unconditionally determined and can appropriately be determined while taking into consideration various factors such as the kinds of triterpenes, the purpose of application such as prophylactic or therapeutic use, the ingestion period, the amount, the age, sex and body weight of a subject who uses the agent, the manner of ingestion such as direct oral administration or the use of the agent as a raw material and/or the intensity of the effect required. For instance, the effective component is used in an amount of not less than 0.00001% by mass, preferably not less than 0.0001% by mass, more preferably 0.001 to 99.9% by mass, further preferably 0.001 to 99.99% by mass, further preferably 0.01 to 99.9% by mass, further preferably 0.01 to 99.99% by mass, further particularly preferably 0.1 to 99.9% by mass, further particularly preferably 0.1 to 99.99% by mass, further particularly preferably 1 to 99.99% by mass, further particularly preferably 2 to 99.99% by mass and most preferably 3 to 99.99% by mass. In this respect, the higher the content of the effective component, the stronger the whitening effect attained, but when directly ingesting the agent through the oral route, it is necessary to control the content while taking into consideration the influence thereof on the human body. On the other hand, when the agent is used as a raw material, it preferably comprises the effective component in a relatively high concentration.

When using maslinic acid, erythrodiol, uvaol, betulinic acid, betulin and/or physiologically acceptable salts or derivatives thereof as raw materials for orally administered whitening agents, these compounds are preferably used in an amount of not less than 0.1% by mass, more preferably 0.1 to 99.99% by mass, further preferably 1 to 99.99% by mass, further preferably 2 to 99.99% by mass, further preferably 10 to 99.99% by mass, further preferably 30 to 99.99% by mass, further preferably 50 to 99.99% by mass, further preferably 70 to 99.99% by mass, further preferably 80 to 99.99% by mass and further preferably 90 to 99.99% by mass.

Moreover, the amount of the 5-membered ring-containing triterpenes and physiologically acceptable salt or derivatives thereof included in the food, beverage or orally administered whitening agent of the present invention required for suitably achieving the whitening effect thereof may vary depending on various factors such as the manner of ingestion, and sex, body weight and physical conditions of the subject who ingests the product and is not restricted to any specific range, but the amount is, for instance, not less than 0.0001 g/day, preferably not less than 0.001 g/day, more preferably not less than 0.01 g/day, particularly preferably not less than 0.1 g/day, further preferably not less than 0.5 g/day, further preferably not less than 1 g/day and most preferably not less than 2 g/day.

The orally administered whitening agent of the present invention is characterized in that it comprises a 5-membered ring-containing triterpene and may be used in various applications, but it may be used in wide variety of fields such as drugs and quasi-drugs. In this respect, the amount of the orally administered whitening agent of the present invention to be incorporated into the foregoing products is not unconditionally be determined since it may vary depending on various factors such as applications, the routes of administration, and the kinds, ages, sexes, body weights, the degrees of symptoms and health conditions of subjects to which the foregoing products are administered, but the amount thereof should of course be one effective for the prevention and/or treatment of the dark skin, liver spots, ephelis, dullness and dark area of the skin.

As has already been discussed above, the orally administered whitening agent of the present invention can be incorporated into foods and beverages to obtain such products having whitening effects. Examples of such foods and beverages to which the orally administered whitening agent of the present invention can be applied have been listed above. The content of the orally administered whitening agent in these foods and beverages may appropriately be determined depending on the intensity of the whitening effect of each orally administered whitening agent selected, and the desired or intended intensity of the whitening effect. The content of at least one member selected from the group consisting of 5-membered ring-containing triterpenes and physiologically acceptable salts or derivatives thereof to be incorporated into the foods and beverages may appropriately be adjusted while using the content thereof in the foregoing foods and beverages as an indication.

The dosage forms of, for instance, drugs, quasi-drugs and beauty and health goods to which the orally administered whitening agent of the present invention is applied are not restricted to specific ones and they may, for instance, be drugs for internal use such as tablets, granules, capsules and aqueous solutions. These drugs may be formed into a desired unit dosage form together with physiologically acceptable additives such as vehicles, carriers, excipients, binders, stabilizers and flavors. For instance, a composition for preparing a tablet or capsule may be admixed with additives, for instance, a binder such as tragacanth, Arabic gum, gelatin, hydroxypropyl cellulose and calcium carboxymethyl cellulose; an excipient such as crystalline cellulose, microcrystalline cellulose, saccharides (e.g., lactose, sucrose and glucose) and starch (e.g., corn, potato and wheat starches); a swelling agent such as gelatinized starch and alginic acid; a lubricant such as magnesium stearate; a sweetening agent such as sucrose, lactose and saccharin; and a flavor such as peppermint, oil derived from G. adenothrix Maxim. and cherry and then formed into each desired dosage form in accordance with the usual method.

The orally administered whitening agent of the present invention may likewise be ingested as such without any pre-treatment or may be incorporated into orally ingested products such as foods, beverages and drugs as a raw material.

The present invention relates to a method for using, as an orally administered whitening agent, at least one member selected from the group consisting of oleanane type triterpenes such as maslinic acid and erythrodiol, ursane type triterpenes such as ursolic acid and uvaol, lupane type triterpenes such as betulinic acid and betulin and physiologically acceptable salts or derivatives thereof. In this respect, the term "use" means, as has been described above, the use of these compounds as a prophylactic agent and/or a therapeutic agent for eliminating or relieving the dark skin, liver spots, ephelis, dark area and dullness of the skin and includes both the use thereof as raw materials and the direct use thereof as orally administered drugs.

The present invention relates to a food or beverage and an orally administered whitening agent, which comprise 5-membered ring-containing triterpenes and physiologically acceptable salts or derivatives thereof. These 5-membered ring- containing triterpenes are excellent, in particular, in the effect of whitening the skin and therefore, the food, beverage or orally administered whitening agent of the invention is quite preferred since one can favorably enjoy the effect of whitening the skin easily and continuously without requiring any burden such as time and labor. In addition, the 5-membered ring-containing triterpenes used in the invention are preferred since they can be obtained from naturally occurring plants, they can be used in daily life without any risk and they may give users mentally refreshing feeling.

### Examples

The present invention will hereunder be described in more detail with reference to the following Examples, but the present invention is not restricted to these specific Examples at all.

As to the 5-membered ring-containing triterpenes used in the following Examples, the following triterpenes are purchased as reagents: erythrodiol (Funakoshi Co., Ltd.), ursolic acid (Wako Pure Chemical Co., Ltd.), uvaol (Funakoshi Co., Ltd.), betulinic acid (Funakoshi Co., Ltd.) and betulin (Funakoshi Co., Ltd.). In this respect, those of HPLC grade were used without any pre-treatment and the other triterpenes were dissolved in ethanol heated to its boiling point to the saturation point, followed by the recrystallization through cooling, filtration and drying prior to practical use. As will be detailed below with reference to experiments, maslinic acid used herein was obtained by extracting the same from olive plant, purifying it to a purity of 95%.

### Preparation Example 1

Dry fruits (including seeds; 500 g) of home growing olive plant (Olea europaea L.) were crushed and 3 L of hexane was added to the crushed fruits to carry out extraction for 3 hours. The seeds were removed from the defatted fruits (defatted products) obtained after repeating the foregoing extraction procedure over 4 times, followed by the pulverization of the defatted products and additional extraction with 5 volumes of hexane for 3 hours to thus obtain 229 g of defatted products from which the oil components were completely removed. To the defatted products, there were added 10 volumes of an aqueous ethanol solution having an ethanol content of 60% by mass and the extraction was continued at room temperature for 3 hours with vigorous agitation. The whole system was filtered and the resulting filtrate was concentrated to dryness to give 112.7 g of an extracted product.

To 100 g of this extracted product, there was added 21 of water and the mixture was vigorously stirred at room temperature for one hour. The whole mixture was centrifuged, the resulting supernatant was removed through decantation and the resulting precipitates were dried to give 10.0 g of a concentrate.

Then the concentrate was fractionated by silica gel column chromatography using a column packed with about 40 volumes of silica gel (400 g). First, an eluent (3: 1 hexane/ethyl acetate mixed solvent) was passed through the column in an amount of about 10 times (4000 mL) the volume of the silica gel packed in the column to remove various kinds of undesirable fractions and further 2.5 volumes (1000 mL) of an eluent (1: 1 hexane/ethyl acetate mixed solvent) was passed through the column to likewise remove various kinds of undesirable fractions. Subsequently, 10 volumes (4000 mL) of an eluent (1: 1 hexane/ethyl acetate mixed solvent) were passed through the column to thus elute desired maslinic acid and to give a crude maslinic acid fraction. After the hexane and ethyl acetate were removed from the fraction, the resulting residue was vacuum dried to give 1.96 g of a crude maslinic acid-containing fraction.

Moreover, the resulting crude maslinic acid fraction was purified by the ODS column chromatography using a column packed with about 30 volumes (60 g) of octadecyl silica gel. First, miscellaneous undesirable components were eluted by passing an eluent (8: 2 methanol/water mixture) through the column in an amount of 10 times (600 mL) the volume of the packed silica gel. Then intended maslinic acid was eluted by passing an eluent (8: 2 methanol/water mixture) through the column in an amount of 30 times (1800 mL) the volume of the packed silica gel to give a purified maslinic acid fraction. After removal of the methanol from this fraction, the resulting residue was vacuum-dried to give 1.51 g of purified maslinic acid 1.

The purified maslinic acid 1 was analyzed by, for instance, the NMR and MS spectroscopic measurements and as a result, it could be confirmed that a part of the maslinic acid included in the fraction was in the form of salts such as sodium and potassium salts and that the majority of the remaining portion was in its free acid form. Moreover, the purities of these substances were evaluated based on GC measurements and it was confirmed that the purity of maslinic acid was not less than 95%.

### Preparation Example 2

To 1 kg of oil expression residue (oil expression products) obtained after the oil expression of olive (Olea europaea L.) of Italian growth, there were added 10 volumes of an aqueous ethanol solution having an ethanol content of 65% by mass, followed by extraction at room temperature for 3 hours with vigorous agitation. After filtering the whole system, the resulting filtrate was concentrated to dryness to give 20.2 g of an extract.

To this extract, there were added 1 L of n-butanol and 1 L of water, the resulting mixture was stirred for 10 minutes and then divided into an n-butanol phase and an aqueous phase. After removing the n-butanol in the n-butanol phase, the residue was vacuum-dried to give 13.3 g of a concentrate.

Then the concentrate was fractionated by silica gel column chromatography using a column packed with about 40 volumes of silica gel (500 g). First, an eluent (3: 1 hexane/ethyl acetate mixture) was passed through the column in an amount of about 10 times (5000 mL) the volume of the silica gel packed in the column to remove various kinds of undesirable fractions and further 2.5 volumes (1250 mL) of an eluent (1: 1 hexane/ethyl acetate mixture) was passed through the column to likewise remove various kinds of undesirable fractions. Subsequently, 10 volumes (5000 mL) of an eluent (1: 1 hexane/ethyl acetate mixture) were passed through the column to thus elute desired maslinic acid and to give a crude maslinic acid fraction. After the hexane and ethyl acetate were removed from the fraction, the resulting residue was vacuum dried to give 2.66 g of a crude maslinic acid-containing fraction.

Moreover, the crude maslinic acid fraction was purified by the ODS column chromatography using a column packed with about 30 volumes (80 g) of octadecyl silica gel. First, miscellaneous undesirable components were eluted by passing an eluent (8: 2 methanol/water mixture) through the column in an amount of 10 times (800 mL) the volume of the packed silica gel. Then intended maslinic acid was eluted by passing an eluent (8: 2 methanol/water mixture) through the column in an amount of 30 times (2400 mL) the volume of the packed silica gel to give a purified maslinic acid fraction. After removal of the methanol from this fraction, the resulting residue was vacuum-dried to give 2.06 g of purified maslinic acid 2.

The purified maslinic acid 2 was analyzed by, for instance, the NMR and MS spectroscopic measurements and as a result, it could be confirmed that a part of the maslinic acid included in the fraction was in its free acid form and that the majority of the remaining portion was in the form of salts such as sodium and potassium salts. Moreover, the purities of these substances were evaluated based of GC measurements and it was confirmed that the purity of maslinic acid was not less than 97%.

### Preparation Example 3

To 1 kg of the extraction residue derived from olive of Italian growth prepared in the olive oil-manufacture process (defatted products obtained by further subjecting oil expression residues to an extraction process), there were added 10 volumes of ethanol and then the mixture was heated to 55°C to carry out extraction with vigorous stirring for 3 hours. After filtering the whole system, the resulting filtrate was concentrated to dryness to give 35 g of an extracted product.

Then the extracted product was subjected to silica gel column chromatography using a column packed with about 40 volumes of silica gel (1400 g). First, an eluent (3: 1 hexane/ethyl acetate mixture) was passed through the column in an amount of about 10 times (14 L) the volume of the silica gel packed in the column to remove various kinds of undesirable fractions and further 2.5 volumes (3500 mL) of an eluent (1: 1 hexane/ethyl acetate mixture) was passed through the column to likewise remove various kinds of undesirable fractions. Further, 10 volumes (14 L) of an eluent (1: 1 hexane/ethyl acetate mixture) were passed through the column to thus elute desired maslinic acid and to give a crude maslinic acid fraction. After the hexane and ethyl acetate were removed from the fraction, the resulting residue was vacuum dried to give 5.90 g of a crude maslinic acid-containing fraction.

Moreover, the crude maslinic acid fraction was purified by the ODS column chromatography using a column packed with about 30 volumes (180 g) of octadecyl silica gel. First, miscellaneous undesirable components were eluted by passing an eluent (8: 2 methanol/water mixture) through the column in an amount of 10 times (1800 mL) the volume of the packed silica gel. Then intended maslinic acid was eluted by passing an eluent (8: 2 methanol/water mixture) through the column in an amount of 30 times (5400 mL) the volume of the packed silica gel to give a purified maslinic acid fraction. After removal of the methanol from this fraction, the resulting residue was vacuum-dried to give 5.36 g of purified maslinic acid 3.

The purified maslinic acid 3 was analyzed by, for instance, the NMR and MS techniques and as a result, it could be confirmed that a part of the maslinic acid included in the fraction was in its free acid form and that the majority of the remaining portion was in the form of salts such as sodium and potassium salts. Moreover, the purities of these substances were evaluated on the basis of GC measurements and it was confirmed that the purity of maslinic acid was not less than 97%.

Derivatives of 5-membered ring-containing triterpenes were prepared as follows.

### Synthetic Example 1: Ethyl Maslinate

Maslinic acid (4.5 g) and triethylamine (1.0 g) were dissolved in 50 mL of chloroform and the resulting mixture was stirred for one hour while dropwise adding a solution of 1.1 g of thionyl chloride in 10 mL of chloroform with ice cooling. Subsequently, 3.2 g of ethanol was added to the mixture and the resulting mixture was stirred for 3 hours while dropwise adding a solution of 1.0 g of triethylamine in 10 mL of chloroform with ice cooling. After the completion of the reaction, the chloroform-soluble moiety was extracted and the chloroform was distilled off to give a crude reaction product, followed by purification through silica gel chromatography to give 3.5 g of maslinic acid ethyl ester.

### Synthetic Example 2: 2,3-O-di-Acetyl-maslinic acid

Maslinic acid (2.0 g) was dissolved in 100 mL of pyridine, 50 mL of acetic anhydride was added to the resulting solution and the mixture was stirred overnight. After the pyridine and acetic anhydride were distilled off, the residue was dissolved in ether, the ether phase was washed once with a IN aqueous hydrochloric acid solution, once with an aqueous saturated sodium bicarbonate solution and three times with pure water, magnesium sulfate was added to the mixture and the mixture was allowed to stand overnight. The magnesium sulfate was removed through filtration, the ether was distilled off from the filtrate to give a crude reaction product, and the latter was purified by silica gel column chromatography to give 2.2 g of 2,3-O-di-acetyl-maslinic acid.

### Synthetic Example 3: 2,3 -O-di-Triethylsilyl-maslinic acid triethylsilyl ester

Maslinic acid (1.0 g) was dissolved in 200 mL of anhydrous dimethylformamide, 144.0 mg of imidazole and 350 µ L of triethylsilyl chloride were added to the solution at 0°C, the reactor was hermetically sealed and the content thereof was stirred for 2 hours. After distilling off the dimethylformamide, the resulting residue was dissolved in ether, the ether phase was washed once with a IN aqueous hydrochloric acid solution, once with an aqueous saturated sodium bicarbonate solution and three times with pure water, magnesium sulfate was added to the mixture and the resulting mixture was allowed to stand overnight. The magnesium sulfate was removed through filtration, the ether was distilled off from the filtrate to give a crude reaction product, and the latter was purified by silica gel column chromatography to give 1.5 g of 2,3-O-di-triethylsilyl-maslinic acid triethylsilyl ester.

### Synthetic Example 4: 2,3-O-di-Stearoyl-maslinic acid ethyl ester

The maslinic acid (1.0 g) obtained in Synthetic Example 1 was dissolved in 50 mL of anhydrous toluene, 5.0 g of triethylamine was added to the resulting solution, the resulting mixture was stirred for one hour while 6.0 g of stearic acid chloride was gradually added to the mixture with ice cooling and the mixture was further stirred for 9 hours while the temperature of the mixture was gradually brought back to room temperature. A sufficient amount of a IN HCl aqueous solution was added to the mixture, extraction was carried out with ether, the resulting ether phase was further washed once with a saturated aqueous solution of sodium bicarbonate and three times with pure water, magnesium sulfate was added to the mixture and the resulting mixture was allowed to stand overnight. The magnesium sulfate was removed through filtration, the ether was distilled off from the filtrate to give a crude reaction product, and the latter was purified by silica gel column chromatography to give 1.2 g of 2,3-O-di-stearoyl-maslinic acid ethyl ester.

### Synthetic Example 5: 3,28-O-di-Acetyl-erythrodiol

Erythrodiol (5.0 g) was dissolved in 250 mL of pyridine, 100 mL of acetic acid anhydride was added to the resulting solution and the mixture was stirred overnight. After the pyridine and acetic acid anhydride were distilled off, the resulting residue was dissolved in ether, the ether phase was washed once with a IN aqueous HCl solution, once with an aqueous saturated sodium bicarbonate solution and three times with pure water, magnesium sulfate was added to the mixture and the mixture was allowed to stand overnight. The magnesium sulfate was removed through filtration, the ether was distilled off from the filtrate to give a crude reaction product, and the latter was purified by silica gel column chromatography to give 5.4 g of 3,28-O-di-acetyl-erythrodiol.

### Synthetic Example 6: Ursolic acid ethyl ester

Ursolic acid (5.0 g) and triethylamine (1.1 g) were dissolved in 50 mL of chloroform and the resulting solution was stirred for one hour while dropwise adding a solution of 1.2 g thionyl chloride in 10 mL of chloroform with ice cooling. Then 3.5 g of ethanol was added and the resulting mixture was stirred for 3 hours, while dropwise adding a solution of 1.1 g of triethylamine in 10 mL of chloroform with ice cooling. After the completion of the reaction, the chloroform-soluble moiety was extracted, the chloroform was distilled off from the resulting extract to give a crude reaction product and the latter was purified by silica gel column chromatography to give 3.8 g of ursolic acid ethyl ester.

### Synthetic Example 7: 3,28-O-di-Acetyl-Uvaol

Uvaol (5.0 g) was dissolved in 250 mL of pyridine, 100 mL of acetic anhydride was added to the resulting solution and the mixture was allowed to stand overnight. After the pyridine and acetic anhydride were distilled off, the residue was dissolved in ether, the resulting ether phase was washed once with a IN aqueous HCl solution, once with an aqueous saturated sodium bicarbonate solution and three times with pure water, magnesium sulfate was added to the mixture and the mixture was allowed to stand overnight. The magnesium sulfate was removed through filtration, the ether was distilled off from the filtrate to give a crude reaction product, and the latter was purified by silica gel column chromatography to give 5.4 g of 3,28-O-di-acetyl-uvaol.

### Synthetic Example 8: Betulinic acid ethyl ester

Betulinic acid (5.0 g) and triethylamine (1.1 g) were dissolved in 50 mL of chloroform and the solution was stirred for one hour while dropwise adding a solution of 1.2 g of thionyl chloride in 10 mL of chloroform with ice cooling. Then 3.5 g of ethanol was added to the resulting mixture and the resulting mixture was stirred for 3 hours, while dropwise adding a solution of 1.1 g of triethylamine in 10 mL of chloroform with ice cooling. After the completion of the reaction, the chloroform-soluble moiety was extracted, the chloroform was distilled off from the resulting extract to give a crude reaction product and the latter was purified by silica gel column chromatography to give 3.8 g of betulinic acid ethyl ester.

### Synthetic Example 9: 3,28-O-di-Acetyl-Betulin

Betulin (5.0 g) was dissolved in 250 mL of pyridine, 100 mL of acetic anhydride was added to the solution and the mixture was stirred overnight. After the pyridine and acetic anhydride were distilled off, the residue was dissolved in ether, the resulting ether phase was washed once with a IN aqueous HCl solution, once with an aqueous saturated sodium bicarbonate solution and three times with pure water, magnesium sulfate was added to the mixture and the mixture was allowed to stand overnight. The magnesium sulfate was removed through filtration, the ether was distilled off from the filtrate to give a crude reaction product, and the latter was purified by silica gel column chromatography to give 5.4 g of 3,28-O-diacetyl-betulin.

### Example 1: Evaluation of Melanin Production-Inhibitory Function

A culture medium (2 mL/well) was dispensed to a 6-well plate, a predetermined amount of B-16 melanoma cells was inoculated into the culture medium in each well and the cells were cultured at 37°C and 5% CO₂ by allowing the culture medium to stand. On the next day, a solution of test sample (each 5-membered ring-containing triterpene to be evaluated) having a predetermined concentration was added to and mixed with the culture medium and the cultivation was continued. On the 5^{th} day from the initiation of the cultivation, the culture medium was exchanged and the test sample solution was again added to each well. On the next day, the culture medium was removed from each well to thus recover the cells, followed by washing the cells with PBS (phosphate-buffered physiological saline) and evaluation of the desired function based on the degree of whiteness of the cells. In this connection, the melanin production- inhibitory effect was evaluated by comparing the resulting degree of whiteness with that observed when 450 ppm of vitamin C-magnesium phosphate having a known effect was added instead of the test sample solution (positive control) and that observed when any test sample was not added (control) according to the following criteria.

The evaluation criteria for the degree of whiteness of cells are as follows:

| Rank | Evaluation Standard |
|---|---|
| ++ | The degree of whiteness is higher than that observed for the positive control. |
| + | The degree of whiteness is almost identical to that of the positive control. |
| ± | The degree of whiteness is not higher than that observed for the positive control, but higher than that observed for control. |
| ― | The degree of whiteness is almost identical to that observed for the control. |

The melanin production-inhibitory function was evaluated in accordance with the foregoing method. The results thus obtained are listed in the following Table 1.

**Table 1:**

| Results of Evaluation of Melanin Production-Inhibition | | | | | | | | |
|---|---|---|---|---|---|---|---|---|
| | Concentration (ppm) | | | | | | | |
| | 2 | 4 | 6 | 8 | 10 | 15 | 25 | 50 |
| Purified maslinic acid 1 | ± | + | ++ | ++ | ++ | | | |
| Purified maslinic acid 2 | ± | + | ++ | ++ | ++ | | | |
| Purified maslinic acid 3 | ± | + | ++ | ++ | ++ | | | |
| Erythrodiol | ± | | ± | | ± | | + | + |
| Ursolic acid | ± | + | ++ | ++ | ++ | | | |
| Uvaol | - | | - | | + | | + | + |
| Betulinic acid | - | | ± | | + | | + | + |
| Betulin | - | | ± | | ± | | + | + |
| Compound of Synthetic Example 1 | ± | + | ++ | ++ | ++ | | | |
| Compound of Synthetic Example 2 | ± | ± | + | ++ | ++ | ++ | | |
| Compound of Synthetic Example 3 | - | | ± | | + | | + | + |
| Compound of Synthetic Example 4 | ± | ± | + | ++ | ++ | ++ | | |
| Compound of Synthetic Example 5 | - | | ± | | ± | | + | + |
| Compound of Synthetic Example 6 | ± | + | ++ | ++ | ++ | | | |
| Compound of Synthetic Example 7 | - | | - | | ± | | + | + |
| Compound of Synthetic Example 8 | - | | ± | | ± | | + | + |
| Compound of Synthetic Example 9 | - | | - | | ± | | ± | + |

**Table 1 (continued)**

| | Concentration (ppm) | | | | | | | |
|---|---|---|---|---|---|---|---|---|
| | 10 | 15 | 25 | 50 | 100 | 200 | 300 | 450 |
| Standard: Vitamin C-Mg Phosphate | - | - | - | - | - | ± | ± | + |
| Control: Kojic acid | - | - | - | ± | ± | + | ++ | ++ |
| *: Standard is the degree of whiteness observed when 450 ppm of vitamin C-magnesium phosphate was added. | | | | | | | | |

The data listed in Table 1 clearly indicate that when the melanin production- inhibitory functions of 5-membered ring-containing triterpenes and physiologically acceptable salts or derivatives thereof are compared with that observed when 450 ppm of vitamin C-magnesium phosphate is added (positive control), all of the compounds tested have melanin production-inhibitory functions several ten times to several hundred times that observed for the positive control (450 ppm). For instance, the function observed for free maslinic acid (purified maslinic acid 1) at a concentration of 4 ppm is identical to that observed for 450 ppm of the positive control. This means that free maslinic acid (purified maslinic acid 1) has a melanin production-inhibitory function about 110 times higher than that observed for the positive control (vitamin C-magnesium phosphate). Similarly, when comparing with the result obtained using 450 ppm of vitamin C-magnesium phosphate (standard), it can be confirmed that the melanin production-inhibitory function is about 110 times (a maslinic acid salt; purified maslinic acid 2), about 20 times (erythrodiol), about 110 times (ursolic acid), about 40 times (uvaol), about 40 times (betulin), about 20 times (betulinic acid), about 110 times (ethyl maslinate), about 75 times (acetylated maslinic acid), about 40 times (triethylsilylated maslinic acid), about 75 times (stearoyl-maslinic acid ethyl ester), about 20 times (acetylated erythrodiol), about 110 times (ursolic acid ethyl ester), about 20 times (acetylated uvaol), about 20 times (ethyl betulinate) and about 10 times (acetylated betulin) that observed for the standard.

### Example 2: Animal Test for the Confirmation of Whitening Effect

Brown guinea pigs (5-week-old male animals) were preliminarily kept for one week using a commercially available feed (CG-7 available from Nippon Kurea Company), a half of the hair on the back was removed and the hair-removed portion on the back was irradiated with light rays emitted from a UV lamp of 1.2 mW for 5 minutes on the 1^{st} day and then with light rays emitted from a UV lamp of 1.0 mW for 5 minutes on the 2^{nd} and 3^{rd} days. Subsequently, these animals were kept over 2 weeks using a feed having a low vitamin C content (200.0 mg/kg feed), these guinea pigs were divided into 12 groups (comprising 7 animals each) in such a manner that there was not observed any difference in the degree of deposition of melanin pigment on the skin induced by the UV light irradiation between groups and then the animals were allowed to freely ingest each test feed comprising the feed having a low vitamin C content to which each triterpene had been added in an amount specified in the following Table 2. In this respect, however, the vitamin C-deficient feed per se was supplied to the control group and a vitamin C-supplemented feed was supplied to the positive control group. After 4 weeks from the supply of the test feed, the degree of the pigmentation of the skin was evaluated according to the following criteria. The results thus obtained are summarized in the following Table 3.

**Table 2:**

| Composition of Feed | | |
|---|---|---|
| Animal Group No. | Additive | Added Amt. (mg/kg feed) |
| 1 (Control) | -- | -- |
| 2 (Positive Control) | L-Ascorbic acid | 2500.0 |
| 3 | Purified maslinic acid 1 | 500.0 |
| 4 | Purified maslinic acid 1 | 250.0 |
| 5 | Erythrodiol | 500.0 |
| 6 | Ursolic acid | 500.0 |
| 7 | Uvaol | 500.0 |
| 8 | Betulinic acid | 500.0 |
| 9 | Betulin | 500.0 |
| 10 | Purified maslinic acid 2 | 500.0 |
| 11 | Compound of Synthetic Ex. 1 | 500.0 |
| 12 | Compound of Synthetic Ex. 2 | 500.0 |

| (Evaluation Criteria) | |
|---|---|
| Rank | Evaluation Standard |
| Conspicuo us Imp. (CI) | There was observed pigmentation clearly lower than that observed for the positive control group. |
| Medium Imp. (MI) | There was observed pigmentation almost identical to that observed for the positive control group. |
| Slight Imp. (SI) | The pigmentation observed was intermediate between those observed for the control and positive control groups. |
| No Change (NC) | There was observed pigmentation almost identical to that observed for the control group. |

**Table 3:**

| Evaluation of Whitening Effect Observed on Guinea Pigs | | | | |
|---|---|---|---|---|
| Test Animal Group No. | Evaluation Results | | | |
| | CI | MI | SI | NC |
| 1 (Control) | 0 | 0 | 0 | 7 |
| 2 (Positive Control) | 0 | 1 | 4 | 2 |
| 3 | 4 | 3 | 0 | 0 |
| 4 | 2 | 3 | 2 | 0 |
| 5 | 2 | 2 | 2 | 1 |
| 6 | 4 | 2 | 1 | 0 |
| 7 | 1 | 4 | 1 | 1 |
| 8 | 2 | 4 | 1 | 0 |
| 9 | 1 | 3 | 2 | 1 |
| 10 | 3 | 3 | 1 | 0 |
| 11 | 2 | 3 | 1 | 1 |
| 12 | 2 | 4 | 1 | 0 |

As has been indicated in Table 3, the 5-membered ring-containing triterpenes and physiologically acceptable salts or derivatives thereof permit the improvement of the skin pigmentation in its early stage and can make such skin pigmentation inconspicuous by the oral administration of these compounds.

| **Example 3:** Edible Oil and Fat Preparation | |
|---|---|
| Purified maslinic acid 1 of Preparation Example 1 | 5.0 g |
| Soybean oil | 1000.0 g |

Purified maslinic acid 1 was added to soybean oil in a rate specified above and these components were sufficiently mixed and dissolved till the whole system became clear using a stirring machine while maintaining the temperature of the system at 60°C to thus form an edible oil and fat preparation.

| **Example 4:** Dressing | |
|---|---|
| Water | 46.6 (g) |
| Xanthan gum | 0.1 |
| Fructose, glucose, liquid sugar | 5.0 |
| Common salt | 5.0 |
| MSG | 0.3 |
| Rice vinegar (acid value: 10%) | 10.0 |
| Pepper | As much as suffices (q.s.) |
| Purified maslinic acid 2 of Preparation Ex. 2 | 1.0 |
| Soybean salad oil | 32.0 |

The raw materials other than the soybean salad oil were introduced into a container equipped with a stirring machine and capable of being warmed in a rate specified in the foregoing table, heated till the temperature of the mixture reached 90°C while stirring the same at 100 rpm using a propeller stirrer and the mixture was further stirred for 25 minutes while maintaining the temperature of the mixture at 90°C. Thereafter, the mixture was cooled down to 20°C and blended with the soybean salad oil to give a dressing. The resulting dressing was found to have good taste and texture.

| **Example 5:** Tablet-Like Sweet | |
|---|---|
| Citric acid | 1.0 (g) |
| Skimmed milk powder | 15.5 |
| Sucrose fatty acid ester | 1.0 |
| Flavor | q.s. |
| Purified maslinic acid 1 of Preparation Example 1 | 2.5 |
| Granulated sugar | 20.0 |
| Lactose | 60.0 |

The foregoing raw materials were uniformly admixed, granulated and compressed into tablet-like sweet (500 mg each).

| **Example 6:** Cookies | |
|---|---|
| Margarine | 70.0 (g) |
| Sugar | 40.0 |
| Common salt | 0.7 |
| Whole egg | 20.0 |
| Soft flour | 100.0 |
| 3,28-O-di-Acetyl-uvaol of Preparation Example 7 | 0.1 |

The raw materials were admixed in the mixing ratio specified above, the resulting mixture was divided into pieces (10 g each) and the pieces were baked at 180°C for 15 minutes to give cookies.

| **Example 7:** Refreshing Drink | |
|---|---|
| 3,28-O-di-Acetyl-erythrodiol of Synthetic Example 5 | 0.5 (g) |
| Honey | 15.0 |
| Citric acid | 0.1 |
| dl-Malic acid | 0.1 |
| D-Sorbitol solution (70%) | 10.0 |
| Sodium benzoate | 0.1 |
| Perfume | As much as suffices. |
| Purified water | ad. 100 g |

The foregoing raw materials were uniformly admixed to give a health drink.

| **Example 8:** Tablet | |
|---|---|
| Purified maslinic acid 1 prepared in Preparation Example 1 | 250.0 (mg) |
| Corn starch | 14.5 |
| Crystalline cellulose | 25.0 |
| Calcium carboxymethyl cellulose | 10.5 |

The raw substances were sufficiently admixed in the mixing ratio specified above and the resulting mixture was compressed into a tablet (300 mg each).

| **Example 9:** Edible Oil and Fat Preparation | |
|---|---|
| Purified maslinic acid 3 prepared in Preparation Example 3 | 10.0 (g) |
| EXV Olive oil | 1000.0 |

To the EXV (extra virgin) olive oil, there was added the purified maslinic acid 3 in a rate specified above and these components were sufficiently admixed and dissolved using a stirring machine till the whole system became clear while maintaining the temperature of the system at 60°C to thus form an edible oil and fat preparation.

| **Example 10:** Margarine | |
|---|---|
| Rape seed oil | 42.0 (g) |
| Hardened rape seed oil | 42.0 |
| Water | 14.0 |
| Common salt | 0.5 |
| Lecithin | 0.5 |
| Monoglyceride | 0.4 |
| Purified maslinic acid 1 prepared in Preparation Example 1 | 0.6 |
| Perfume | q.s. |
| Carotene | Trace amt. |

The foregoing raw materials were admixed together according to the usual method and the resulting mixture was subjected to a quenching-kneading treatment using a combination machine to give margarine.

| Example 11: Mayonnaise | |
|---|---|
| Soybean salad oil | 74.0 (g) |
| Water | 8.4 |
| Sugar | 1.0 |
| Sodium glutamate | 0.3 |
| Powdery mustard | 0.3 |
| Common salt | 1.0 |
| Rice vinegar | 4.0 |
| Purified maslinic acid 2 prepared in Preparation _Example 2 | 1.0 |
| Egg yolk to which common salt is added (salted egg yolk) | 10.0 |

The foregoing raw materials except for soybean salad oil and salted egg yolk in a mixing ratio specified above were heated to 90°C while mixing and stirring them and the mixture was stirred for 25 minutes while maintaining the temperature thereof at 90°C. After cooling the mixture down to 20°C, it was combined with the soybean salad oil and salted egg yolk and the resulting mixture was stirred under reduced pressure to give mayonnaise.

| **Example 12:** Powder | |
|---|---|
| Purified maslinic acid 2 prepared in Preparation Example 2 | 10.0 (mg) |
| Lactose | 981.0 |
| Hydroxypropyl cellulose | 4.0 |
| Light anhydrous silicic acid | 5.0 |

First, purified maslinic acid 2 and lactose were sufficiently admixed together, hydroxypropyl cellulose was added to the resulting mixture and then the mixture was granulated. After drying the granulated mixture, the particle size of the mixture was adjusted, the light anhydrous silicic acid was added and they were sufficiently admixed to thus give a powder.

| **Example 13:** Capsule | |
|---|---|
| Ursolic acid ethyl ester of Synthetic Example 6 | 150.0 (mg) |
| Lactose | 70.0 |
| Corn starch | 38.0 |
| Magnesium stearate | 2.0 |

The ingredients were sufficiently admixed in a mixing ratio specified above and then the resulting mixture was encapsulated to give capsules.

### Example 14: Test for Confirming the Whitening Effect on Human Body

Regarding the whitening effect of the tablet-like sweet prepared in Example 5, the tablet was practically ingested to evaluate the degree of improvement or relief of the dark skin, liver spots, ephelis, dark area and dullness of the skin according to the following method.

### (Test Method)

This test was carried out using, as subjects, 30 common women of 25 to 50-year-old who suffered from or troubled with the dark skin, liver spots, ephelis, dark area and dullness of the skin. More specifically, these 25 to 50-year-old women (30 persons) were randomly divided into two sections (15 women each) or a control section and a test section and the persons of the control section were requested to taste 3 tablet-like sweets similar to the tablet-like sweet (500 mg each) of Example 5 and free of any 5-membered ring-containing triterpene and the persons of the test section were requested to have a foretaste of 3 tablet-like sweets (500 mg each) prepared in Example 5, simultaneous with each diet (breakfast, lunch and supper) or 9 tablets (4.5 g) in all per day. The tasting or sampling was continued over 12 weeks and at this stage, we got each subject to self-evaluate the degrees of the improvement in the dark skin, liver spots, ephelis, dark area and dullness of the skin in accordance with the following criteria. The results thus obtained are listed in the following Table 4.

| (Evaluation Criteria) | |
|---|---|
| Rank | Evaluation Standard |
| Conspicuo us Imp. (CI) | The dark skin, liver spots, ephelis, dark area and dullness of the skin could be almost inconspicuous by the ingestion of the test sample. |
| Medium Imp. (MI) | The dark skin, liver spots, ephelis, dark area and dullness of the skin could be considerably inconspicuous by the ingestion of the test sample. |
| Slight Imp. (SI) | The dark skin, liver spots, ephelis, dark area and dullness of the skin could somewhat be inconspicuous by the ingestion of the test sample. |
| No Change (NC) | The conditions of the skin were not improved as compared with those observed before the ingestion of the test sample. |

**Table 4**

| | CI | MI | SI | NC |
|---|---|---|---|---|
| Dark Skin: Control section | 0 | 0 | 2 | 13 |
| Test section 5 | 5 | 6 | 4 | 0 |
| Liver spots, ephelis: Control | 0 | 0 | 1 | 14 |
| section Test section | 7 | 5 | 3 | 0 |
| Dark Area of the Skin: Control | 0 | 1 | 4 | 12 |
| section Test section | 3 | 7 | 4 | 1 |
| Dullness of the Skin: Control | 0 | 1 | 3 | 11 |
| section Test section | 4 | 5 | 4 | 2 |

As indicated by the results shown in Table 4, the ingestion of the tablet-like sweets of Example 5 would permit the prevention and relief of the dark skin, liver spots, ephelis, dark area and dullness of the skin and make these symptoms inconspicuous and make the skin beautiful.

The food or beverage and orally administered whitening agent of the present invention permit the easy and continuous ingestion of 5-membered ring-containing triterpenes and physiologically acceptable salts or derivatives thereof without any burden such as time and labor and accordingly, one can favorably enjoy, in particular, the effect of whitening the skin. Moreover, the 5-membered ring-containing triterpenes can be isolated from natural substances and therefore, one can use the same without any fear.

## Claims

1. A food or beverage for whitening the skin or an orally administered whitening agent comprising, as an effective component, a compound selected from the group consisting of 5-membered ring-containing triterpenes and physiologically acceptable salts or derivatives thereof.

2. The food or beverage for whitening the skin or orally administered whitening agent of claim 1, wherein the 5-membered ring-containing triterpenes and physiologically acceptable salts thereof are those isolated from naturally occurring substances.

3. The food or beverage for whitening the skin or orally administered whitening agent of claim 1, wherein the content of the 5-membered ring-containing triterpenes and physiologically acceptable salts or derivatives thereof is not less than 0.04% by mass on the basis of the total mass of the food or beverage for whitening the skin or orally administered whitening agent.

4. The food or beverage for whitening the skin or orally administered whitening agent of claim 1, wherein the 5-membered ring-containing triterpenes are compounds selected from the group consisting of oleanane type triterpenes, ursane type triterpenes and lupane type triterpenes.

5. The food or beverage for whitening the skin or orally administered whitening agent of claim 1, wherein the 5-membered ring-containing triterpene is selected from the group consisting of maslinic acid, erythrodiol, ursolic acid, uvaol, betulinic acid and betulin.

6. The food or beverage for whitening the skin or orally administered whitening agent of claim 1, wherein the derivative of 5-membered ring-containing triterpene is an alcohol ester group-containing or fatty acid ester group-containing derivative.

7. The food or beverage of claim 1, wherein the food or beverage is a processed food.

8. The food or beverage of claim 7, wherein the processed food is an oil and fat preparation or an oil and fat-processed product.

9. The food or beverage of claim 8, wherein the oil and fat-processed product is margarine, shortening, mayonnaise or dressing.

10. The food or beverage of claim 1, wherein the food or beverage is a refreshing drink.

11. The food or beverage of claim 1, wherein a part or the whole of the 5-membered ring-containing triterpenes are present in the form of physiologically acceptable salts and/or derivatives thereof.

12. An edible oil and fat preparation comprising not less than 1% by mass of maslinic acid obtained by extracting defatted products of olive with an ethanol solution, concentrating the resulting extract through drying and then purifying the resulting concentrate by chromatography.

13. A dressing comprising not less than 1% by mass of maslinic acid obtained by extracting oil expression products derived from olive with an ethanol solution, concentrating the resulting extract through drying and then purifying the resulting concentrate by chromatography.

14. A tablet-like sweet comprising not less than 2.5% by mass of maslinic acid obtained by extracting defatted products of olive with an ethanol solution, concentrating the resulting extract by drying and then purifying the resulting concentrate by chromatography.

15. A food or beverage comprising the orally administered whitening agent as set forth in claim 1.

16. An orally administered whitening agent comprising maslinic acid obtained by extracting defatted products of olive with an ethanol solution, concentrating the resulting extract by drying and then purifying the resulting concentrate by chromatography and in the form of a tablet.

17. An orally administered whitening agent comprising maslinic acid obtained by extracting defatted products of olive with an ethanol solution, concentrating the resulting extract by drying and then purifying the resulting concentrate by chromatography and in a form of powder.

18. A method of using, as an orally administered whitening agent, a compound selected from the group consisting of maslinic acid, erythrodiol, ursolic acid, uvaol, betulinic acid, betulin and physiologically acceptable salts or derivatives thereof.

19. Use of the orally administered whitening agent as set forth in claim 1 as prophylactic and/or therapeutic agents for dark skin, liver spots, ephelis, dark area of the skin and dullness of the skin.
